Europäisches Patentamt

European Patent Office

Office européen des brevets:

(19)

(11) Publication number: **0 153 437**
**A1**

(12)

# EUROPEAN PATENT APPLICATION

(21) Application number: **84105779.7**

(22) Date of filing: **21.05.84**

(51) Int. Cl.⁴: **G 08 C 15/00**
**A 61 B 5/00, G 01 N 27/28**
**H 03 F 3/72**

(30) Priority. **24.05.83 DK 2327/83**

(43) Date of publication of application:
**04.09.85 Bulletin 85/36**

(84) Designated Contracting States:
**AT BE CH DE FR GB IT LI LU NL SE**

(71) Applicant. **RADIOMETER A/S**
**Emdrupvej 72**
**DK-2400 Copenhagen NV(DK)**

(72) Inventor: **Krogh, Soren-Christian**
**Baertoften 1 Ganlose**
**DK-2760 Malov(DK)**

(74) Representative: **Patentanwälte Grünecker, Dr.**
**Kinkeldey, Dr. Stockmair, Dr. Schumann, Jakob, Dr.**
**Bezold, Meister, Hilgers, Dr. Meyer-Plath**
**Maximilianstrasse 58**
**D-8000 München 22(DE)**

(54) Electrochemical measuring electrode device and method for transmission of signals therefrom.

(57) In an electrochemical measuring electrode device (10) comprising sensor means (12, 13 and 36) for generation of one or more measuring signals, the measuring signals are multiplexed in a first multiplexing means included in an integrated electronic circuit (23) and transmitted therefrom through a two conductor cable (37) to a cooperating measuring apparatus (50). In the measuring apparatus (50), the measuring signals are demultiplexed and displayed on a display (48). The circuit (23) is mounted on an insulating ceramic substrate (21) also supporting a temperature dependent NTC resistor (28) and a Zener diode (30) together constituting a thermostating means of the electrode device. The electronic circuit of the electrode device (10) and the heat generating Zener diode (30) are supplied with power from the measuring apparatus (50). The transmission is preferably carried out in time-division multiplexed state, so that the measuring results generated by means of the sensor means and the temperature representative signal generated by the NTC resistor (28) are transmitted within individual time intervals, while an electrical power signal for supply of the electronic circuitry of the electrode device and for supply of the Zener diode (30) is transmitted within a specific sub-period of each of the transmission time intervals.

The gated amplifier comprises a high-gain differential DC-amplifier a storage capacitor and a switch means for conversion of the gated amplifier to a mode in which any input offset of the DC-amplifier is transmitted in a unity gain mode.

Fig. 1.

## ELECTROCHEMICAL MEASURING ELECTRODE DEVICE AND METHOD FOR TRANSMISSION OF SIGNALS THEREFROM

---

### BACKGROUND OF THE INVENTION

The present invention relates to a method of transmission of measuring signals from an electrochemical measuring electrode device to measuring equipment.

Hitherto, when transmitting measuring signals generated in an electrochemical measuring electrode device, the signals have been transmitted individually through individual cores of a multicore cable connecting the electrode device and the measuring equipment to one another. Conventionally, the multicore cable is at one end attached to the electrode device and at the other end provided with a multipin plug. The measuring equipment is provided with a mating socket for receiving the multipin plug. Although the employment of a multicore cable connection is in itself a very simple technique, it does, however, suffer from some serious drawbacks. Firstly, the multicore cable, and the multipin plug and cooperating socket may be extremely expensive, especially when the number of signals is large, as the number of individual cores in the multicore cable and the number of pins in the multipin plug and socket become correspondingly large. Secondly, the process in which a large number of individual cores of a multicore cable is soldered onto a multipin plug and onto soldering terminals or soldering pins of the electrode device, is rather delicate and consequently a time-consuming and expensive operation. Thirdly, the multicore cable and the multipin plug may be rather bulky. Fourthly, as a consequence of the large number of individual cores, pins and soldered connections, the entire system is liable to mechanical failure. If a failure occurs, one or more of the signals transmitted from the electrode device to the measuring equipment may be influenced, and in some cases totally interrupted, and in other cases the transmission is only partially interrupted so that the measuring equipment may receive erroneous signals which are interpreted as measuring signals and consequently falsely read out. A further serious problem involved in multicore cable transmission is the problem associated with crosstalk between the individual cores caused by capacitive or inductive coupling therebetween. The elimination of crosstalk may be highly complicated and expensive as the problem may only be solved by employing specially constructed, highly complex cables, which, as mentioned above, may be very expensive.

It is therefore an object of the present invention to provide a method of transmission of measuring signals from an electrochemical measuring electrode device to measuring equipment, in which the above-mentioned drawbacks and problems are eliminated and solved.

SUMMARY OF THE INVENTION

In a first aspect, the present invention provides a method of transmission of measuring signals from an electrochemical measuring electrode device to measuring equipment, the electrode device comprising sensor means adapted to generate at least two individual measuring signals, and the measuring signals being transmitted from the electrode device to the measuring equipment through a cable, wherein, prior to transmission through the cable, the measuring signals are multiplexed in a first multiplexer means, and wherein, after transmission through the cable, the signals are demultiplexed in a second multiplexer means.

By transmission of measuring signals from the electrode device to the measuring equipment in a multiplexed state, it is rendered possible to employ a simple two-conductor cable, e.g. a coaxial cable, for the transmission, and, consequently, the above problems and drawbacks associated with multicore cables are solved and eliminated.

Multiplex transmission is a well-known technique in the art. However, it has hitherto not been employed for transmission of measuring signals from an electrochemical measuring electrode device to measuring equipment. Multiplex transmission is normally carried out either in the so-called frequency or frequency-division multiplexing mode or in the so-called time or time-division multiplexing mode. By frequency multiplexing, the individual signals are multiplexed having a specific carrier and transmitted within specific frequency bands where the signals are demultiplexed by separating the signals from the carrier. In time multiplexing, each of the individual signals is transmitted one at a time and within individual time intervals so that within a first time interval a first signal is transmitted, within a second time interval a second signal is transmitted and so forth so that all signals are transmitted within a so-called multiplexing cycle, whereupon the sequential transmission is repeated.

In accordance with the present invention, the measuring signals are preferably transmitted in a time multiplexed state so that the measuring signals are transmitted from the first multiplexer means to the second multiplexer means within specific time intervals of a multiplexing cycle. By carrying out the transmission in time multiplexed state, it is rendered possible to transmit DC-signals directly without encoding the measuring signals, i.e. transferring the measuring signals from analogue to e.g. digital form.

When measuring signals are transmitted from an electrochemical measuring electrode device, especially an electrode device adapted to carry out physiological measurements, the variations of the measuring signals are normally relatively slow so that the time multiplexing cycle may have a relatively low frequency of approximately 10 Hz-1 kHz, preferably approximately 100 Hz.

Since the electrode means may be of either current generating or voltage generating nature and may generate measuring signals within different ranges, i.e. within different current ranges or voltage ranges, it is preferred that the measuring signals are normalized prior to multiplexing. In the present context, the term "normalized" means that the measuring signals are tranferred into voltage signals supplied from voltage generators of approximately identical voltage generating capacity. Especially by the transfer of measuring results generated by current generators, e.g. generated in polarographic electrode systems, into voltage signals supplied from a low impedance output, it is rendered possible to transmit the measuring signals from the first multiplexer means to the second multiplexer means over a long distance without causing high frequency losses or noise pick-up.

When the measuring signals are normalized prior to multiplexing, it is preferred that the normalization is carried out in offset compensated amplifier means so that any inherent offset of the amplifier means is compensated so that the offset of the amplifier means do not contribute to the normalized measuring signals supplied therefrom.

When transmitting measuring signals from the electrode device to the measuring equipment in a multiplexed state, the first multiplexer means has to be supplied with electrical power. Although the electrode device may itself include a power supply, e.g. a battery supply, the electrical power may, in accordance with the present invention, be transmitted from said second multiplexer means to said first multiplexer means for supply thereof so that a common power supply contained in the measuring equipment is employed for powering the first multiplexer means.

When the measuring signals are transmitted from said first multiplexer means to said second multiplexer means in a time multiplexed state, the electrical power for supplying the first multiplexer means may be transmitted from said second multiplexer means to said first multiplexer means within a specific time interval of the multiplexing cycle.

Although the transmission of measuring signals from the first multiplexer means to the second multiplexer means may be controlled in any appropriate manner, e.g. controlled by the second multiplexer means, it is preferred that the transmission of measuring signals from the first multiplexer means to the second multiplexer means is controlled by a voltage controlled oscillator of said first multiplexer means, i.e. the transmission is inherently controlled by the first multiplexer means. By employing a voltage controlled oscillator of the first multiplexer means for controlling the transmission therefrom, it is rendered possible to adjust the multiplexing cycle by simply adjusting the voltage component of the electrical power transmitted from the second multiplexer means to the first multiplexer means. Thus, any interference from external noise generating sources may be eliminated. Furthermore, by increasing the transmission frequency, the so-called 1/f noise of the amplifier means may be reduced.

In order to synchronize the first multiplexer means and the second multiplexer means relative to one another, a synchronizing signal which is derived from the electrical power supplied from the second multiplexer means to the first multiplexer means may be transmitted from the first multiplexer means to the second multiplexer means.

Apart from the measuring signals which are transmitted from the first multiplexer means to the second multiplexer means, and the electrical power supply signals which are transmitted from the second multiplexer means to the first multiplexer means, the electrode device may further be connected in a control or feedback loop so that a measuring signal is transmitted from the electrode device to the measuring equipment and a control signal is transmitted from the measuring equipment to the electrode device in response to the said measuring signal. In accordance with the present invention, when measuring signals are transmitted from an electrochemical measuring electrode device which further comprises thermostating means including temperature sensor means and temperature controlling means, a temperature signal representative of the temperature detected by the temperature sensor means may be multiplexed in the first multiplexer means and transmitted therefrom to the second multiplexer means within a specific time period of the multiplexing cycle and supplied to the measuring equipment, and a temperature controlling signal generated in the measuring equipment, in response to the temperature representative signal, may be multiplexed in the second multiplexer means and transmitted therefrom to the first multiplexer means within another specific time period of the multiplexing cycle, and supplied to the temperature controlling means for activation thereof.

Normally, the electrode device merely includes a single temperature sensor means and a single temperature controlling means together constituting a single thermostating means, but the measuring electrode device may also comprise a first and a second thermostating means including a first and a second temperature sensor means and a first and a second temperature controlling means in accordance with the principle described in EP Patent Application No. 82109436.4, publication No. 0 077 073 and International Patent Application No. PCT/DK82/00093, publication No. WO 83/01510. In order to provide transmission to and from the first and the second thermostating means of the electrode device, temperature signals representative of the temperatures detected by the first and the second temperature sensor means may in accordance with the present invention be multiplexed in the first multiplexer means and transmitted therefrom to the second multiplexer means within a first and a second specific time period of the multiplexing cycle, respectively, and be supplied to said measuring equipment, while temperature controlling signals generated in the measuring equipment, in response to the temperature representative signals, may be multiplexed in the second multiplexer means and transmitted therefrom to the first multiplexer means within a third and a fourth specific time period of the multiplexing cycle and supplied to the first and the second temperature controlling means, respectively, for activation thereof.

When measuring signals and temperature representative signals are transmitted from the electrode device to the measuring equipment, and electrical power and temperature controlling signals, in response to the temperature representative signals, are transmitted from the measuring equipment to the electrode device, the temperature controlling signal or signals transmitted from the second multiplexer means to the first multiplexer means may, in accordance with the present invention, be constituted by the current component of the power which is transmitted from the second multiplexer means to the first multiplexer means within the above mentioned specific time interval of the multiplexing cycle.

In another aspect, the present invention provides a method of transmission of at least one measuring signal from an electrochemical measuring electrode device to measuring equipment through a cable, the electrode device comprising sensor means adapted to generate said one or more measuring signals and a first multiplexer means, and the measuring equipment comprising a second multiplexer means, wherein said one or more measuring signals are multiplexed in said first multiplexer means and are transmitted therefrom through the cable to said second multiplexer means and demultiplexed therein, and wherein an electrical power signal for supply of said electrode device is multiplexed in said second multiplexer means and is transmitted therefrom through the cable to said first multiplexer means and demultiplexed therein.

In accordance with this particular aspect of the present invention, it is rendered possible to transmit at least one measuring signal, e.g. a single measuring signal, from the electrochemical measuring electrode device to the measuring equipment while eliminating the above-mentioned drawbacks. A particular advantage of this special aspect of the present invention is the elimination of the above mentioned transmission distance limitations and noise pick-up drawbacks associated with current and/or voltage generating electrode means. A further advantage is the possibility of obtaining low-noise, normalized measuring signals from different kinds of measuring devices, i.e. a standardization of the general measuring equipment may be obtained.

In accordance with a further aspect, the present invention provides a method of transmission of at least one measuring signal from an electrochemical measuring electrode device, which is thermostatically heated, to measuring equipment through a cable, the electrode device comprising sensor means adapted to generate said one or more measuring signals, thermostating means including temperature sensor means and temperature controlling means, and a first multiplexer means, and the measuring equipment comprising a second multiplexer means, wherein said one or more measuring signals together with a signal representative of the temperature detected by said temperature sensor means are multiplexed in said first multiplexer means and are transmitted therefrom through the cable to said second multiplexer means and demultiplexed therein, and wherein an electrical power signal for supply of said electrode device together with a temperature controlling signal is multiplexed in said second multiplexer means and is transmitted therefrom through the cable to said first multiplexer means and demultiplexed therein.

P&V F3100B jA, HSN/IP, 1984 05 10/1

In accordance with the present invention, the measuring signals may, prior to multiplexing, be converted from analogue form to digital form in an A/D-converter, and, if desired, after transmission through the cable, be converted from digital form to analogue form in a D/A-converter. However, in the most simple embodiment of the invention, the measuring signals are transmitted in analogue form from the first multiplexer means to the second multiplexer means and, if desired, after transmission through the cable, the measuring signals are converted from analogue form to digital form in an A/D-converter prior to demultiplexing in the second multiplexer means so that a digital representation of the normalized measuring signals are output from the second multiplexer means. Thus, digital calculation equipment, e.g. a microcomputer, may be connected directly to the output of the second multiplexer means for receiving the measuring signals in digital form for processing in the microcomputer.

The present invention also relates to an electrochemical measuring electrode device which comprises sensor means adapted to generate at least two individual measuring signals, and which is adapted to be connected through a cable to corresponding measuring equipment and to cooperate therewith for transmission of said measuring signals from the electrode device to the measuring equipment, wherein the electrode device further comprises first multiplexer means, which is connected to said electrode means and is adapted to provide said measuring signals, which are to be transmitted to said measuring equipment, in a multiplexed state, and to cooperate with corresponding second multiplexer means of said measuring equipment, said second multiplexer means being adapted to provide said transmitted measuring signals in a demultiplexed state.

In another aspect, the present invention provides an electrochemical measuring electrode device which comprises at least one sensor means adapted to generate at least one measuring signal, and which is adapted to be connected through a cable to corresponding measuring equipment and to cooperate therewith for transmission of said one or more measuring signals from the electrode device to the measuring equipment, wherein the electrode device further comprises first multiplexer means connected to said electrode means and adapted to cooperate with corresponding second multiplexer means of said measuring equipment, wherein said first multiplexer means is adapted to provide said one or more measuring signals to be transmitted to said measuring equipment in a multiplexed state, said second multiplexer means being adapted to provide said transmitted one or more measuring signals in a demultiplexed state, and wherein said first multiplexer means is further adapted to receive an electrical power supply signal for supply of the electrode device from said second multiplexer means.

In a further aspect, the present invention provides a thermostatically heated electrochemical measuring electrode device which comprises at least one sensor means adapted to generate at least one measuring signal and thermostating means including temperature sensor means and temperature controlling means, and which is adapted to be connected through a cable to corresponding measuring equipment and to cooperate therewith for transmission of said one or more measuring signals from the electrode device to the measuring equipment, wherein the electrode device further comprises first multiplexer means connected to said electrode means and adapted to cooperate with corresponding second multiplexer means of said measuring equipment, wherein said first multiplexer means is adapted to provide said one or more measuring signals to be transmitted to said measuring equipment in a multiplexed state, said second multiplexer means being adapted to provide said transmitted one or more measuring signals in a demultiplexed state, wherein said first multiplexer means is further connected to the temperature sensor means and the temperature controlling means, and is adapted to transmit a signal representative of the temperature detected by the temperature sensor means to said second multiplexer means of the measuring equipment, and to receive an electrical power supply signal for supply of the electrode device and a temperature controlling signal generated in the measuring equipment, in response to the temperature representative signal, from said second multiplexer means, and wherein said first multiplexer means is further adapted to supply said electrical power supply signal to the electrode device and to supply said temperature controlling signal to said temperature controlling means for activation thereof.

A particular aspect of the present invention relates to a gated amplifier means for connection to an external measuring signal generating source and to external measuring equipment, comprising a high-gain differential DC-amplifier having an output, an inverting input and a non-inverting input, a storage capacitor connected to one of the inputs of the DC-amplifier and to a DC-supply source for supply of an input offset compensation voltage to the said one input of the DC-amplifier, and a switch means for conversion of the gated amplifier means from a first mode in which an input signal supplied from the signal generating source is amplified in the DC-amplifier and supplied from the output thereof to the measuring equipment, to a second mode in which any input offset of the DC-amplifier is transmitted in a unity gain mode from the gated amplifier means to the measuring equipment and vice versa. By employing a gated amplifier means, an almost perfect input offset compensation may be carried out by measuring the input offset of the DC-amplifier in the second mode and by storing an input offset compensation voltage at the storage capacitor for compensating any input offset of the DC-amplifier. A further advantage of the gated amplifier means is that the so-called 1/f noise, i.e. noise, which is band-limited within the frequency range 1-10 Hz, is almost perfectly compensated for. Furthermore, the so-called pop corn noise, i.e. noise caused by DC-variations or noise having a frequency below 1 Hz, is also almost perfectly compensated for together with any input DC offset of the DC-amplifier.

In a particular embodiment of the gated amplifier means, the switch means comprises a first switch interconnected between the output of the DC-amplifier and the inverting input thereof for converting the DC-amplifier into a unity gain mode, when short-circuiting the output and the inverting input of the DC-amplifier, for transmitting any input offset of the DC-amplifier therethrough and further from the output thereof to the external measuring equipment. By employing a switch interconnecting the output and the inverting input of the DC-amplifier, it is in a very simpel manner rendered possible to convert the gated amplifier means from the said first mode to the said second mode.

0153437

The gated amplifier means may be connected in either an inverting mode, in which the signal generating source is connected to the inverting input of the DC-amplifier, or in a non-inverting mode, in which the signal generating source is connected to the non-inverting input of the DC-amplifier. When the DC-amplifier is connected in a non-inverting mode, the switch means may further comprise a second switch interconnected between the signal generating source and the non-inverting input of the DC-amplifier, and a third switch interconnected between the storage capacitor and the non-inverting input of the DC-amplifier so that the signal generating source and the storage capacitor may alternatively be connected to the non-inverting input of the DC-amplifier.

In the presently preferred embodiment of the gated amplifier means according to the invention, the gated amplifier means comprises a further high-gain differential DC-amplifier connected in a non-inverting mode, the output of said further DC-amplifier being connected to said external measuring equipment, and the switch means comprises a first and a second switch, the first switch being interconnected between the non-inverting input of the further DC-amplifier and the inverting input of the first-mentioned DC-amplifier, and the second switch means being interconnected between the non-inverting input of the further DC-amplifier and the non-inverting input of the first-mentioned DC-amplifier, for transmitting, when the first switch and the second switch, respectively, are activated, any input offset of the inverting input of the first-mentioned DC-amplifier and any input offset of the non-inverting input of the first-mentioned DC-amplifier, respectivly to the further DC-amplifier and further therethrough in a unity-gain, non-inverting mode to the external measuring equipment.

In the preferred embodiment of the gated amplifier means according to the invention, the DC-amplifier is constituted by an operational amplifier. By employing an operational amplifier, a high-gain, high input impedance and low output impedance amplifier is provided.

The electrochemical measuring electrode device according to the invention may be provided as a multicomponent assembly comprising individual or discrete components. In order to obtain a more compact structure, the electrode device may in accordance with the present invention be provided by means of well-known layer techniques, including thick film and thin film techniques, or in accordance with the semiconductor technology, including the so-called ISFET technology. Furthermore, the measuring electrode device according to the invention may be provided in any appropriate combination of discrete components, layer technique and semiconductor technology.

The measurements carried out in accordance with the method according to the invention and by means of the electrochemical measuring electrode device according to the invention comprise measurements for the determination of blood constituents, e.g. the determination of blood gas partial pressures, $Po_2$, or $Pco_2$, or the determination of blood ions, e.g $H^+$, $Li^+$, $Na^+$, $K^+$, $Ca^{++}$, $Mg^{++}$, $HCO_3^-$, and $Cl^-$. The measuring signals generated by means of the sensor means of the electrode device may, apart from signals generated by means of electrochemical sensor means, comprise bio-electrical measuring signals generated by appropriate sensor means, including measuring signals representing ECG (electrocardiography), HBR (heart beat rate), or RR (respiration rate), or any electrical measuring signal representing any physical parameter, e.g. temperature, electrical conductivity, etc.

BRIEF DESCRIPTION OF THE DRAWINGS

The present invention will now be further described with reference to the drawings, wherein
Fig. 1 is a partly sectional view of a first embodiment of an electrochemical measuring electrode device for transcutaneous measurement of the partial pressures of oxygen and carbon dioxide and a measuring apparatus connected and cooperating therewith,
Fig. 2, corresponding to Fig. 1, another embodiment of the electrode device according to the invention,
Fig. 3 a block diagram of an electronic circuit for carrying out the method according to the invention and comprising a transmitter part and a receiver part included in the electrode device and in the measuring apparatus, respectively,

Fig. 4, in greater detail relative to Fig. 3, the transmitter part of the electronic circuit, including a first embodiment of a gated amplifier circuit,

Fig. 5, in greater detail relative to Fig. 3 and corresponding to Fig. 4, the receiver part of the electronic circuit,

Fig. 6, a time base diagram of a single transmission period, and

Fig. 7 an alternative embodiment of one of the gated amplifier circuits, shown in Fig. 4.


DETAILED DESCRIPTION OF THE DRAWINGS


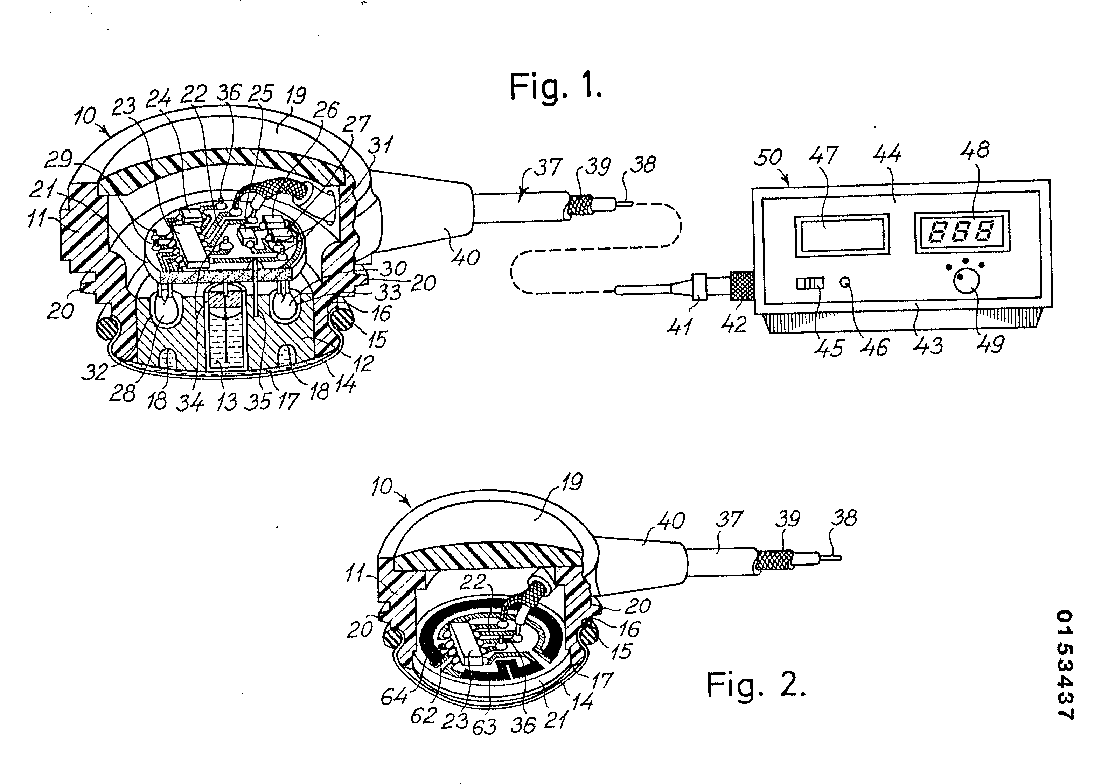

In Figs. 1 and 2, a first and a second embodiment of an electrochemical measuring electrode device according to the invention for transcutaneous measurement of the partial pressures of oxygen and carbon dioxide are shown. The first embodiment shown in Fig. 1 is in its entirety designated by the reference numeral 10. The electrode device 10 comprises an electrode housing 11 in which an Ag-body 12 is embedded. In accordance with well-known measuring principles, vide e.g. US Patent No. 4,058,447, the Ag-body 12 constitutes an anode of a polarographic oxygen electrode system also including a cathode of a noble metal (not shown on the drawing). In accordance with well-known principles, vide e.g. US Patent No. 4,324,256 and International Patent Application No. PCT/DK82/00024, publication No. WO 82/03275, the Ag-body 12 also constitutes a reference electrode for a pH-sensitive electrode 13, the Ag-body 12 and the pH-sensitive electrode 13 together constituting a $Pco_2$-electrode measuring system. In front of the Ag-body 12, a membrane 14 is arranged which is fastened relative to the electrode housing 11 by means of an O-ring 15 received in a circumferential recess 16 of the electrode housing 11. In a space defined between the exposed lower surface of the Ag-body 12 and the membrane 14, an electrolyte solution 17 is enclosed. The membrane and the electrolyte solution may be of the kind described in the above references. Recesses 18 of the Ag-body 12 constitute electrolyte reservoirs. In front of the exposed lower side of the Ag-body 12, a spacer may be arranged. At the top of the housing 11, a cover 19 is arranged, which is adapted to seal the interior space of the electrode device completely. The electrode housing 11 and the cover 19 are preferably made of a plastics material, e.g. acrylonitrile-butadiene-styrene. The housing 11 is further provided with two exterior thread protrusions which are adapted to cooperate with a corresponding thread of a fixation ring for fastening the measuring electrode device therein (not shown on the drawings) also preferably made of acrylonitrile-butadiene-styrene. The fixation ring may be of the kind described in US Patent No. 4,274,418.

In a space defined within the electrode device between the lower side surface of the cover 19 and the upper side surface of the Ag-body 12, a circular board or circular ceramic substrate, e.g. an alumina substrate, 21 is arranged. The board or substrate 21 is at its upper side surface provided with printed circuit conductors 22. On the upper side surface of the board or substrate 21, electronic circuits 23, 24, 25, 26 and 27 are arranged, and through soldering terminals and soldered joints they are arranged in electrically conductive connection with the individual or respective printed circuit conductor 22. At the lower side surface of the circular board or substrate 21, an NTC-resistor 28 is arranged having its pins 29 arranged extending through through-going bores of the circular board or substrate 21 and soldered to two respective printed circuit tracks connected to two respective terminals of the electronic circuit 23 through soldered joints. At the lower side surface of the circular board or substrate 21, a Zener diode 30 is also arranged having its pins 31 extending through through-going bores of the circular board or substrate 21 and soldered to two respective printed circuit tracks connected to respective terminals of the electronic circuits 23, 26 and 27 through soldered joints. As evident from Fig. 1, the components 28 and 30 are embedded in recesses 32 and 33, respectively, at the upper side surface of the Ag-body 12. The components 28 and 30 are fixed in the recesses 32 and 33, respectively, by means of a heat conductive epoxy resin (not shown on the drawing). Together, the NTC-resistor 28 and the Zener diode 30 constitute a thermostating means of the electrode device.

Through a rod 34, which extends through the circular board or substrate 21 and is connected to a printed circuit track 22 through a soldered joint, the above described pH-electrode is connected to a terminal of the electronic circuit 23 through a further soldered joint. Through a rod 35 extending through the circular board or substrate 21, the Ag-body 12 is also connected to the electronic circuit 23 through a printed circuit track 22. Through soldered joints and a printed circuit track 22, the upper end of the rod 36 provides electrical connection from the electronic circuit 23 to the above-mentioned noble metal cathode of the polarographic electrode measuring system.

A two-conductor cable or a coaxial cable 37 has its central conductor 38 and its screen 39 soldered to two respective printed circuit tracks of the circular board or substrate 21 through soldered joints and extends through a hole of the electrode housing 11 and through a stub 40 which protrudes from the electrode housing 11. At its other end, the coaxial cable 37 is provided with a coaxial plug 41 which is adapted to be received in a coaxial socket 42 of an electronic measuring apparatus 50 which is adapted to cooperate with the electrode device 10. The measuring apparatus 50 includes electronic circuitry to be described below and further comprises an apparatus housing 43 and a front plate 44. The apparatus 50, which is preferably a mains-supplied apparatus, is provided with an ON/OFF button 45 and a corresponding ON-indicator lamp 46. A first display 47 is adapted to provide information in case the measuring apparatus or the electrode device is malfunctioning and, consequently, adapted to provide an error indication and information as to what malfunction is present. A second display 48 is adapted to provide a read-out of measuring results obtained by means of the electrode device 10 and determined in the measuring apparatus 50 in alphanumerical manner. A knob 49 is provided for selecting the kind of measuring results displayed on the display 48, i.e. to select measuring results representing the partial pressure of oxygen determined by means of the polarographic electrode system, measuring results representing the partial pressure of carbon dioxide determined by means of the potentiometric electrode system, or measuring results representing the temperature determined by means of the NTC-resistor 28.

0153437

In Fig. 2, a second embodiment of the electrochemical measuring electrode device according to the invention is shown. This second embodiment of the electrode device differs from the embodiment of the invention shown in Fig. 1 in that the circular board or substrate 21, which is preferably a thick film substrate, preferably an alumina substrate, is provided with electrode means at its exposed outer side surface in accordance with the principles described in DK Patent Application No. 1578/80 and patent applications claiming priority therefrom including US Serial No. 253,226 and published German Patent Application No. 31 14 441. In accordance with these principles, the electrode device is at the outer side surface of the substrate 21 provided with a step-like Ag-anode which is connected to a soldering terminal 62 through a metal rivet (not shown on the drawing) embedded in a through-going bore of the substrate. Through a soldered joint, the soldering terminal 62 is connected to a terminal of the electronic circuit 23. The cathode of the polarographic electrode system of the electrode device is constituted by the above described conductor 36 which is preferably a gold or platinum conductor. The conductor 36 is embedded in another through-going bore of the substrate 21 and partly encased in a glass tube as described in the above mentioned DK Patent Application No. 1578/80. The embodiment of the invention shown in Fig. 2 also differs from the embodiment shown in Fig. 1 in that the electrode device is not provided with a pH-electrode. Consequently, the electrode device is not adapted for measurement of the partial pressure of carbon dioxide, but merely for polarographic measurement of the partial pressure of oxygen. However, the embodiment shown in Fig. 2 may quite simply be altered for adaption to measurement of the partial pressures of both oxygen and carbon dioxide by providing a pH-electrode as described in the above-mentioned DK Patent Application No. 1578/80. In the embodiment of the invention shown in Fig. 2, the above thermostating means comprising the NTC-resistor 28 and the Zener diode 30 shown in Fig. 1 is omitted and replaced by a thermostating means comprising a thick film NTC-resistor 63 and a thick film heating resistor 64, respectively. As will be evident from the description above, the electrode device shown in Fig. 2 is adapted to cooperate with the above described measuring apparatus 50 shown in Fig. 1.

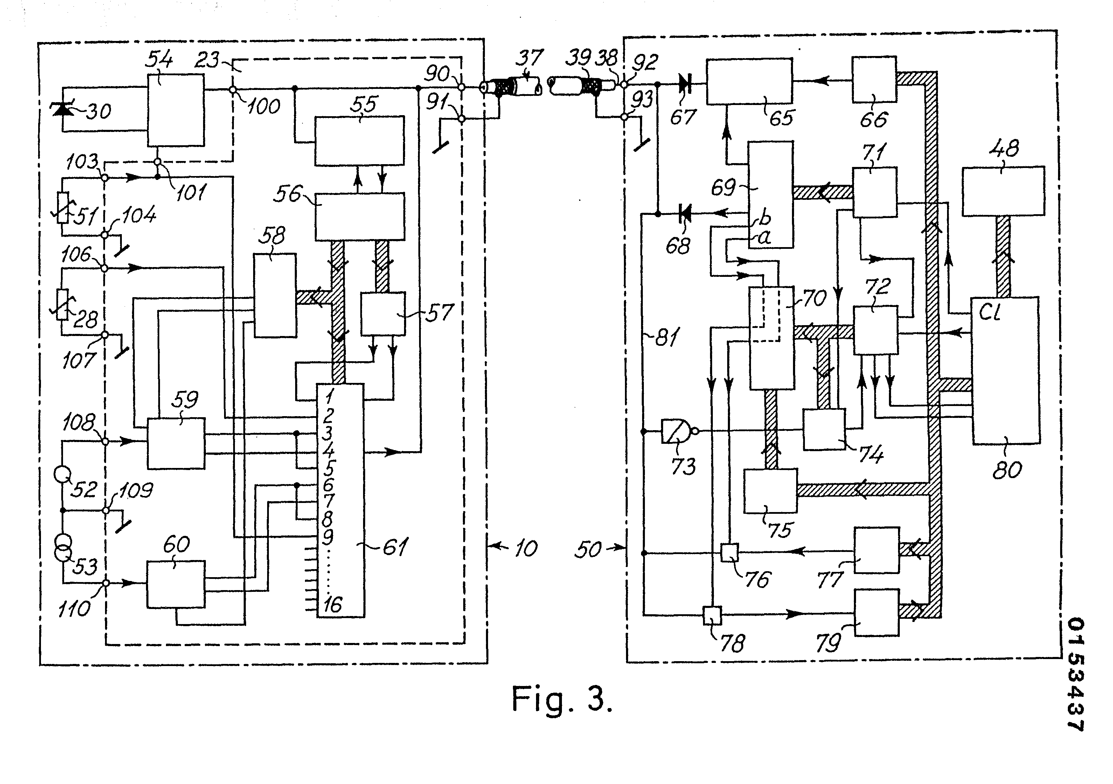

In Fig. 3, a block diagram schematically shows the structure of the electronic circuitry employed in the electrode device 10 and in the measuring apparatus 50. In the present context, the circuit part relating to the electrode device is referred to as the transmitter part and the circuit part relating to the measuring apparatus is referred to as the receiver part. The transmitter and the receiver parts are interconnected by the above coaxial cable 37. The central conductor 38 of the coaxial cable 37 is connected to a hot terminal 90 of the transmitter 10 and to a hot terminal 92 of the receiver 50. Analogously, the screen 39 of the coaxial cable 37 is connected to a cold terminal 91 of the transmitter 10 and to a cold terminal 93 of the receiver 50. Within the transmitter part, which is shown in the left-hand side of Fig. 3, the above electronic circuit 23 is bounded by a dotted line. Through terminals 100 and 101, the electronic circuit 23 is connected to a Zener supply circuit 54 which is further connected to the above Zener diode 30. Apart from the above NTC-resistor 28 which is connected to the circuit 23 through terminals 106 and 107, the circuit 23 is connected to a further NTC-resistor 51, which is connected to the circuit 23 through terminals 103 and 104 and further to the Zener supply circuit 54 through the terminal 101, and which serves the purpose of controlling or checking the temperature so that in case the NTC-resistor 28 fails, the Zener diode 30 is not erroneously activated, which might cause injuries to a patient. As will be explained in greater detail below, the NTC-resistor 51 provides an automatic shut-down of the Zener supply circuit 54, if a predetermined temperature threshold is exceeded. In Fig. 3, the above described potentiometric measuring electrode system comprising the reference electrode 12 and the pH-electrode 13 is designated 52 in its entirety, and the polarographic measuring electrode system comprising the reference electrode or anode 12 and the cathode connected to the rod 36 is designated 53 in its entirety. Through terminals 108, 109 and 110, the electrode systems 52 and 53 are connected to the circuit 23.

The electronic circuit serves two main purposes. Firstly, to transmit measuring results obtained by means of the NTC-resistors 28 and 51, by means of the potentiometric measuring electrode system 52, and by means of the polarographic measuring electrode system 53 to the receiver, i.e. the measuring apparatus 50, in a time-division multiplexed state, and secondly, to receive power from the measuring equipment for supplying the circuit itself and for supplying the Zener supply circuit 54 which further supplies current to the heat generating Zener diode 30. In the time-division multiplexed transmission, a first signal is transmitted within a first time period or transmission period, whereupon a second signal is transmitted within a second time period, and so forth, until all signals, e.g. n signals, have been transmitted. The total transmission time, of e.g. n transmission periods, is called the multiplexing cycle. In the embodiment of the invention preferred at present, the multiplexing cycle has a frequency of 100 Hz. The multiplexing cycle is divided into 16 transmission periods, and each transmission period is further divided into 16 subperiods. During the first 8 subperiods, electrical power of both negative and positive polarity relative to the earth potential of the measuring apparatus 50 is transmitted therefrom to the electrode device 10. During the remaining 8 subperiods of each transmission period, signals, including measuring, compensation, and controlling signals, are transmitted between the transmitter and the receiver. In the embodiment of the invention shown in Fig. 3, the Zener diode 30 is supplied by a negative current, as will be evident from the description below, and the negative supply voltage is transferred from the receiver 50 to the transmitter 10 within the 7 first subperiods of each transmission period, and the positive supply voltage is transferred from the receiver 50 to the transmitter 10 within subperiod No. 8 of each transmission period.

The transmission to and from the transmitter 10 is inherently controlled by a phase locked loop, voltage controlled oscillator 55 which at its output generates an oscillation frequency controlled by the negative voltage supplied thereto through the cable 37. The output of the phase locked loop, voltage controlled oscillator 55 is input to an 8-bit counter 56, which carries out a division of the oscillation frequency supplied from the phase locked loop, voltage controlled oscillator 55 into the individual transmission periods and further into the individual subperiods, and from which the phase locked loop, voltage controlled oscillator 55 receives a comparator signal. The counter 56 is further connected to two decoders 57, and 58, which addresses two gated amplifier circuits 59 and 60, and to an analogue multiplexer 61. A first set of outputs of the counter 56, which is input to the decoder 57, and a second set of outputs of the counter 56, which is input to the decoder 58 and the multiplexer 61, differ in that the first outputs, which are input to the decoder 57, correspond to the above-mentioned subperiods of the individual transmission periods, whereas the second outputs, which are input to the decoder 58 and to the multiplexer 61, correspond to the individual transmission periods. Consequently, the first set of outputs, which is input to the decoder 57, has a frequency which is 16 times higher than the frequency of the second set of outputs.

The gated amplifier circuits 59 and 60 are connected to the potentiometric measuring electrode system 52 and the polarographic measuring electrode system 53, respectively. Together with outputs of the decoder 57 and the NTC-resistors 28 and 51, the outputs of the gated amplifier circuits 59 and 60 are connected to respective inputs of the multiplexer 61. The gated amplifier circuits 59 and 60 at their outputs provide measuring signals generated by means of the respective measuring electrode systems in a normalized state, i.e. within the same voltage signal range from a low impedance output.

A further advantage of the gated amplifier circuits 59 and 60 is that the so-called 1/f noise and the so-called popcorn noise are almost perfectly compensated for.

As explained above, the phase locked loop, voltage controlled oscillator 55 determines the transmission or multiplexing cycle. Apart from controlling the multiplexed transmission, the decoder 57 inputs a synchronizing signal to a first input of the analogue multiplexer 61 in order to synchronize the receiver and the transmitter relative to one another. The synchronizing input signal, which includes several pulses of the subperiod frequency, is transmitted within a first transmission period of the multiplexing cycle, and within a second transmission period of the multiplexing cycle, a temperature signal generated by means of the NTC-resistor 28 is transmitted from the electrode device 10 to the measuring apparatus 50. Within a third transmission period of the multiplexing cycle, the decoder 58 connects the potentiometric measuring electrode system 52 to the input of the gated amplifier circuit 59 so that a voltage generated in the electrode system and amplified in the amplifier circuit 59 is transmitted within this transmission period to the measuring apparatus 50, whereupon within a fourth transmission period, an input offset compensation voltage is retransmitted and stored in a storage device of the gated amplifier circuit 59. Within a fifth transmission period of the multiplexing cycle, the decoder 58 connects the storage device to the input of the gated amplifier circuit 59 so that the input offset compensation voltage is fed through the gated amplifier circuit and further transmitted from the electrode device 10 to the measuring apparatus 50. On the basis of this transmission within the fifth transmission period, the measuring apparatus 50 determines the actual input offset voltage of the gated amplifier circuit 59, and carries out a compensation of the measuring result obtained within the third transmission period. Furthermore, the actual input offset voltage is employed for providing the input offset compensation voltage which is transmitted to the storage device of the gated amplifier circuit 59 within the fourth transmission period of the succeeding transmission cycle. Within a sixth transmission period, the decoder 58 gates the gated amplifier circuit 60 into a current-voltage converter mode for transmission of a signal representing the amplifier input offset voltage and, consequently, the actual polarizing voltage of the polarographic electrode system to the measuring apparatus 50, whereupon within a seventh transmission period, the desired polarizing voltage is retransmitted to the gated amplifier circuit 60. Within an eighth transmission period, the decoder 58 alters the gated amplifier circuit 60 into a high gain mode so that an amplified measuring signal, supplied from a low impedance output and representing the partial pressure of oxygen measured by means of the polarographic electrode measuring system 53 having the desired polarizing voltage, is transmitted from the electrode device 10 to the measuring apparatus 50. Finally, within a ninth transmission period, a temperature signal generated by the NTC-resistor 51 is transmitted from the analogue multiplexer 61 of the electrode device 10 to the measuring equipment 50 through the cable 37. The remaining 7 transmission periods are spare ones and, consequently, only employed for transmission of power from the receiver to the transmitter.

In the right-hand side of Fig. 3, the receiver part of the electronic circuitry is shown. Centrally, the receiver includes a microcomputer 80 which, apart from generating a clock pulse at its CI output, controls the general function of the receiver 50 and furthermore controls and checks the operation of the transmitter 10 as to its transmission and receiving function and its measuring function. The clock frequency generated in the microcomputer 80 is supplied to a first counter 71 which, in a manner known per se, by counting the clock pulses generates a fast counting frequency which corresponds to the frequency of the transmission subperiods, i.e. the frequency of the counting pulses supplied from the counter 56 to the decoder 57. The first counter 71 supplies its fast counting pulses to a second counter 72, which serves the purpose of counting the fast counting pulses for generation of a slow counting frequency corresponding to the frequency of the transmission cycle. The first counter 71 further addresses a first decoding logic 69, and the second counter 72 addresses a second decoding logic 70 and further addresses a synchronizing detector 74 which is connected to the output of a Schmitt-trigger 73. The Schmitt-trigger 73 is at its input connected to a bus bar 81 of the receiver and further to the transmitter 10 through the coaxial cable 37 and is adapted to detect transmission of the synchronizing pulses from the multiplexer 61 of the transmitter 10 within the first transmission period. Each time the Schmitt-trigger 73 detects a synchronizing pulse, the output thereof goes high. In the synchronizing detector 74, the output of the Schmitt-trigger 73 is compared to the fast counting pulses supplied from the first counter 71, and if a predetermined number of synchronizing pulses (in the preferred embodiment, the number is 2) is detected within a first transmission period, the synchronizing detector 74 supplies a synchronizing pulse to the second counter 72, and the synchronism pulse is further transmitted to the microcomputer 80 which also receives the slow counting pulses from the second counter 72.

**0153437**

As explained above, the first counter 71 addresses the first decoding logic 69 by the fast counting pulses. Consequently, the first decoding logic 69 determines the subdivision of the individual transmission period into the above subperiods. Within the first 7 subperiods, the first decoding logic 69 activates a current generator 65 which is controlled by a D/A-converter which is further controlled by the microcomputer 80, and supplies negative current of a predetermined voltage to the bus bar 81 and further to the transmitter 10 through an output diode 67 and through the coaxial cable 37. In the eighth subperiod of each transmission period, the first decoding logic 69 supplies a positive voltage to the bus bar 81 and further to the transmitter 10 through an output diode 68 and through the coaxial cable 37. In the subperiod No. 9, the first decoding logic 69 is pending, while within the subperiods Nos. 10-15, an a-output of the first decoding logic 69 goes high, whereupon within the subperiod No. 16, the first decoding logic 69 is also pending. A further output designated b of the first decoding logic 69 is shifted high within the subperiods 12 and 13. The outputs designated a, b of the first decoding logic 69 are gated through the second decoding logic 70 to a first switch 76 and a second switch 78, respectively. The first switch 76 is interconnected between the bus bar 81 and a D/A-converter 77, and the second switch 78 is interconnected between the bus bar 81 and an A/D converter 79. The D/A converter 77 and the A/D converter 79 are connected to the central microcomputer 80, which is further connected to a register 75, which serves the purpose of sequencing the second decoding logic 70 in accordance with the transmission or multiplexing sequence.

As explained above in connection with the description referring to the transmitter, the first transmission period of the multiplexing cycle is employed for transmission of a synchronizing signal, also referred to above in connection with the Schmitt-trigger 73 and the synchronizing detector 74, whereas the transmission periods 2-9 are employed for transmission of measuring signals from the NTC-resistors 28 and 51 and from the potentiometric and polarographic electrode systems, designated 52 and 53, respectively, and for transmission of the above input offset signals of the gated amplifier circuits 59 and 60 and for retransmission of an input offset voltage compensating signal and an input offset voltage or electrode polarising signal to the amplifiers 59 and 60, respectively. The decoder 57 which controls the activation of the analogue multiplexer 61 within each of the transmission periods, turns on the multiplexer 61 for transmission therefrom or thereto within the subperiods Nos. 11-14.

Contrary to this, the second decoding logic 70 of the receiver 50 gates the b-control signal to the switch 78 within the transmission periods Nos. 2, 3, 5, 6, 8 and 9, and, consequently, turns on the switch 78 for transmission of a signal to the A/D converter 79 within the 12th and 13th subperiod through the switch 78. The A/D converter 79 outputs a digital signal to the central microcomputer 80.

During the transmission periods Nos. 4 and 7, the register 75 addresses the second decoding logic 70 for gating the a-control signal to the switch 76. On the basis of the measuring signals transmitted from the transmitter to the microcomputer 80 of the receiver 50 within the 3rd and the 6th transmission periods, the microcomputer inputs a digital signal to the D/A converter 77 converting the signal into an analogue signal, which is retransmitted through the switch 76 within the subperiods Nos. 10-15 of the transmission periods Nos. 4 and 7 to the bus bar 81 and further through the coaxial cable 37 to the multiplexer 61 of the transmitter 10. On the basis of the temperature representative signal detected by means of the NTC-resistor 28 and transmitted within the subperiods Nos. 10-13 of the second transmission period from the multiplexer 61 of the transmitter 10 to the A/D converter 79 and further to the central microcomputer 80 of the receiver 50 through the coaxial cable 37, the bus bar 81 and the switch 78, the central microcomputer 80 supplies a digital control signal to the D/A converter 66, which converts the signal into an analogue signal representing the current to be supplied within the subperiods Nos. 1-7 of all transmission periods Nos. 1-16, from the current generator 65 to the bus bar 81 and further through the output diode 67, through the coaxial cable 37 and to the Zener supply circuit 54 for supplying and heating the Zener diode 30. Apart from the general control of the receiver 50, the central microcomputer 80 is also connected to the above display 48 for displaying the measuring results selected by means of the selector knob 49 shown in Fig. 1.

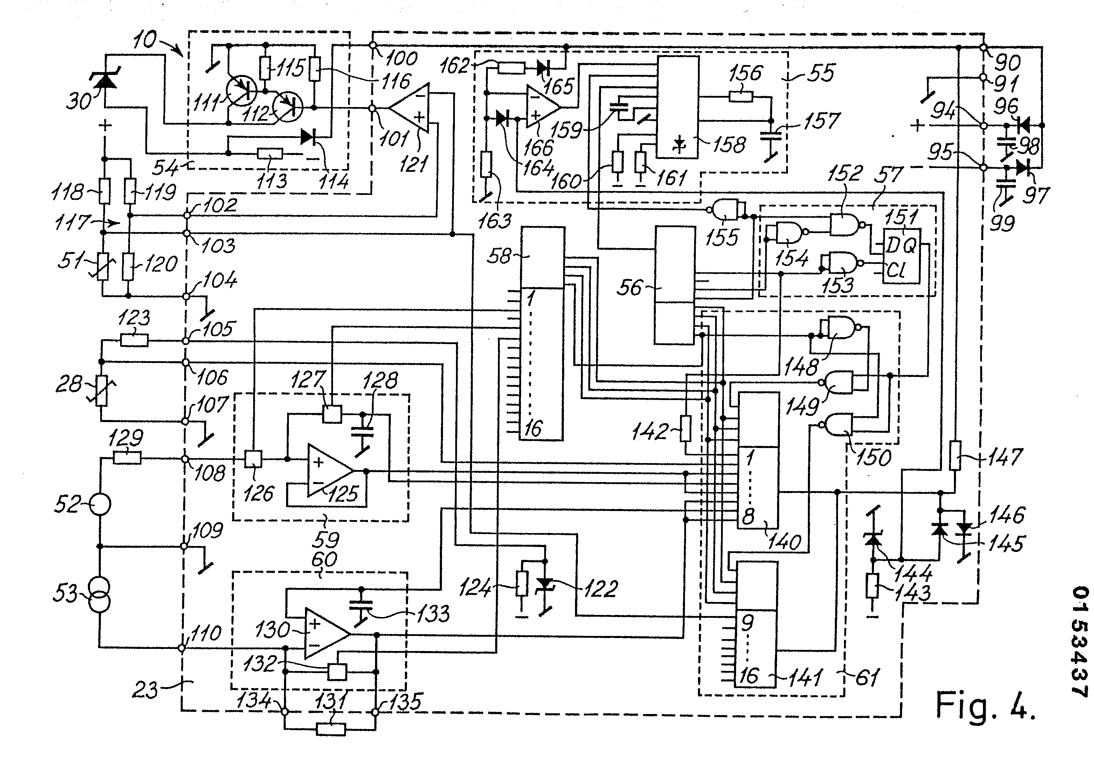

In Fig. 4, the left-hand side of the block diagram shown in Fig. 3, i.e. the transmitter part of the electronic circuitry is shown. In Fig. 4, the blocks of Fig. 3 are indicated by dotted boundary lines. At the top right corner, the hot terminal 90 and the cold terminal 91, which also constitutes the earth terminal of the transmitter 10, are shown. Two more terminals designated 94 and 95 and constituting the positive and the negative supply terminals of the circuit 23, respectively, are connected through diodes 96 and 97, respectively, to the hot terminal 90, and through capacitors 98 and 99, respectively, to the earth terminal of the circuit, i.e. the cold terminal 91. Internally, the positive terminal 94 and the negative terminal 95 are connected to the individual electronic components of the circuit 23 (not shown on the drawing).

At the top left corner of Fig. 4, the Zener supply circuit 54 is shown. The circuit 54 comprises 2 transistors 111 and 112 connected in a Darlington transistor configuration having their common collector connected to the cathode of the Zener diode 30, the anode of which is connected to the negative supply terminal 95 through a resistor 113 and also connected to the hot terminal 90 through a diode 114 and the terminal 100. The bases of the transistors 111 and 112 are connected through resistors 115 and 116, respectively, to the earth or cold terminal 91. The base of the Darlington transistor constituted by the transistors 111 and 112 is connected to the terminal 101 of the circuit 23.

The NTC-resistor 51 which serves the above-mentioned controlling or checking purpose constitutes a variable branch of a conventional Wheatstone bridge 117, further comprising 3 resistors 118, 119 and 120. The Wheatstone bridge 117 is interposed between the positive supply terminal 94 and the terminal 104 which, within the circuit 23, is internally connected to the earth terminal 91. The node of the two resistors 119 and 120 is connected to a non-inverting input of a high gain differential amplifier constituted by an operational amplifier 121, the inverting input of which is connected to the node of the resistor 118 and the NTC-resistor 51. The output of the amplifier 121 is connected to the terminal 101. In case the temperature detected by the NTC-resistor 51 rises, the value of the resistor 51 decreases and, consequently, the potential of the node of the resistor 118 and the NTC-resistor 51 decreases so that the potential at the inverting input of the amplifier 121 decreases. The differential or operational amplifier 121 compares the potential at the non-inverting input, which is a fixed potential defined by the resistors 119 and 120, and the potential at the inverting input, which varies in accordance with the temperature changes influencing the value of the NTC-resistor 51. Consequently, when the potential at the inverting input of the amplifier 121 goes below a certain limit, the output of the amplifier 121 increases beyond the turn-on voltage of the Darlington transistor constituted by the transistors 111 and 112, and, consequently, turns off the Darlington transistor and shuts down the Zener supply circuit.

The NTC-resistor 28 which is connected to the terminals 106 and 107 of the circuit 23 is also connected to a negative voltage reference constituted by a Zener diode 122 through a resistor 123 connected to a terminal 105. The resistor 123 and the NTC-resistor 28 together constitute a voltage divider branch, and, consequently, the potential at the node of the resistor 123 and the NTC-resistor 28 is an indication of the temperature influencing the resistor value of the NTC-resistor 28. The cathode of the Zener diode 122 is connected to the earth terminal 91, and the anode of the Zener diode 22 is connected to the negative supply terminal 95 through a resistor 124.

The gated amplifier circuit 59 which is connected to the potentiometric electrode system 52 through the terminal 108, comprises an operational amplifier 125, which is connected in a non-inverting, unity gain mode, and further two switches 126 and 127, respectively, and a capacitor 128. The switches 126 and 127 are open in their non-activated state. The gates of the switches 126 and 127 are connected to addressing outputs numbered 3 and 5, respectively, of the decoder 58. Consequently, within the 3rd transmission period, the decoder 58 activates the switch 126 and connects the potentiometric electrode

0153437

system 52 through a resistor 129 and the terminal 108 to the non-inverting input of the unity gain amplifier 125, and within the same transmission period, the voltage generated by the potentiometric electrode system 52 is transmitted from the low impedance output of the operational amplifier 125 through the multiplexer 61 and further to the measuring apparatus 50. Within the 4th transmission period, an input offset compensation voltage is transmitted from the receiver 50 to the transmitter 10 and fed from the multiplexer 61 to the capacitor 128 which constitutes the above storage device of the gated amplifier circuit 59 and which is charged. Within the 5th transmission period, the decoder 58 which has its addressing output numbered 5 connected to the gate of the switch 127 activates the switch 127 and, consequently, the voltage of the capacitor 128 is input to the non-inverting input of the operational amplifier 125, the output of which only differs from the input offset compensation voltage by the actual input offset of the amplifier itself. Consequently, on the basis of the output transmitted form the .operational amplifier 125 within the 5th transmission period the measuring apparatus 50 is able to determine exactly the actual input offset of the operational amplifier. The above input offset compensation voltage which is actually identical to the inverted value of the input offset voltage determined within the preceding transmission cycle, may instead have a fixed reference value so that the measuring apparatus 50 determines the input offset voltage of the operational amplifier 125 as the difference between the output of the operational amplifier 125 and the reference voltage of the capacitor 128.

The embodiment of the gated amplifier circuit 60, shown in Fig. 4, which is connected to the polarographic measuring electrode system 53 through the terminal 110, comprises an operational amplifier 130 which is connected in an inverting mode, a feedback resistor 131 which is actually a high precision discrete resistor connected to the inverting input and the output of the operational amplifier 130 through terminals 134 and 135, respectively, a switch 132, which is connected in parallel with the feedback resistor 131 and the gate of which is connected to the addressing output No. 6 of the decoder 58, and a capacitor 133. The operational amplifier 130 inherently shifts its output and, consequently, its inverting input to such a level that its non-inverting and inverting inputs only differ from one another by the input offset voltage of the amplifier. Consequently, the voltage of the capacitor 133 is transferred to the polarographic measuring electrode system 53 and polarises the electrodes to one another. As in the gated amplifier circuit 59, the switch 132 of the gated amplifier circuit 60 is only activated within a single transmission period, viz. the 6th transmission period in which the switch 132 short-circuits the feedback resistor 131, and, consequently, turns the gated amplifier circuit into a unity gain inverting mode or current follower mode for transmission of an output signal from the operational amplifier identical to the actual polarising voltage of the electrode system within the 6th transmission period. On the basis of the actual polarising voltage transmitted within the 6th transmission period and the voltage of the capacitor 133, the measuring apparatus 50 determines the actual input offset of the operational amplifier 130. Within the 7th transmission period, the desired polarising voltage is transferred from the measuring apparatus 50 to the capacitor 133 for biasing the electrodes of the polarographic measuring electrode system 53 relative to one another. Within the 8th transmission period, the current generated by the polarographic measuring electrode system 53 is measured by the operational amplifier 130 which is in a high gain mode, and the output measuring voltage of the operational amplifier 130 is transmitted through the multiplexer 61 and further to the measuring apparatus 50.

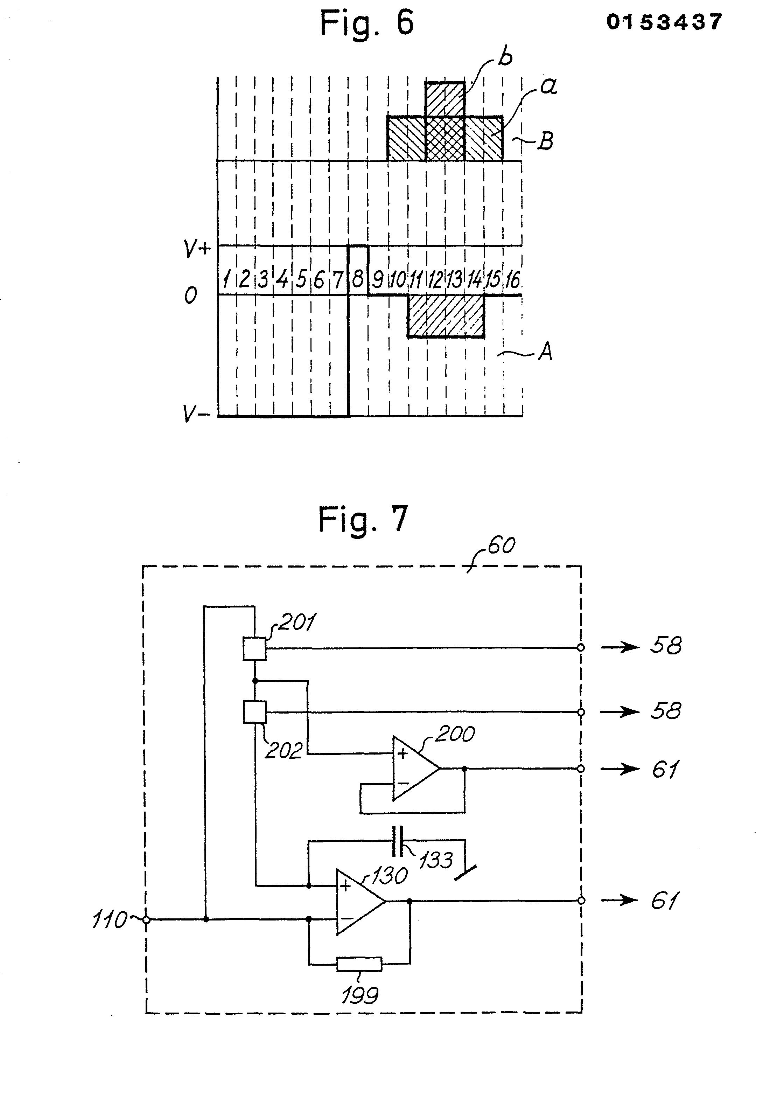

In Fig. 7, an alternative, presently preferred embodiment of the gated amplifier circuit 60 is shown. Apart from the above described operational amplifier 130 and the polarising capacitor 133, the embodiment shown in Fig. 7 includes a feed back resistor 199, basically corresponding to the feed back resistor 131, shown in Fig. 4, which connects the operational amplifier 130 into a high-gain inverting mode, a further operational amplifier 200 which is connected in a unity-gain, non-inverting mode, and a first and a second switch, designated 201 and 202, respectively. The switches 201 and 202 are interconnected between the non-inverting input of the operational amplifier 200 and the inverting input of the operational amplifier 130, and between the non-inverting input of the operational amplifier 200 and the non-inverting input of the operational amplifier 130, respectively. As in the gated amplifier circuit 59, shown in Fig. 4, the gates of the switches 201 and 202 are connected to individual addressing outputs of the decoder 58, actually the addressing outputs Nos. 10 and 11, respectively. The output of the operational amplifier 200 is connected to individual channels, actually channel Nos. 10 and 11 of the multiplexer 61. Consequently, within the 10th transmission period, the decoder 58 addresses the switch 201, and the input offset voltage of the inverting input of the operational amplifier 130 is transmitted through the switch 201 to the non-inverting input of the operational amplifier 200 and further from the output thereof through the multiplexer 61 and through the cable 37 to the measuring apparatus 50 shown in the right hand side of Fig. 3. Similarly, within the 11th transmission period, the decoder 58 addresses the switch 202, and the input offset voltage of the non-inverting input of the operational amplifier 130 is transmitted through the switch 202 to the non-inverting input of the operational amplifier 200 and further from the output thereof through the multiplexer 61 and through the cable 37 to the measuring apparatus 50. Although the operational amplifier 200 in itself adds its input offset voltage to the voltages supplied to the non-inverting input thereof, the input offset voltage of the operational amplifier 200 does not result in erroneous determination of the input offset voltage of the operational amplifier 130 within the measuring apparatus 50, as the measuring apparatus 50 subtracts the voltages supplied thereto within the tenth and eleventh transmission period from one another, and, consequently, eliminates the input offset voltage fault of the operational amplifier 200 when determining the input offset voltage of the operational amplifier 130. Consequently, a highly accurate input offset voltage compensated, gated amplifier circuit 60 is provided. While the embodiment shown in Fig. 4 of the gated amplifier circuit 60 requires three transmission periods, actually the sixth, the seventh and the eighth transmission period, for determining the input offset voltage of the operational amplifier 130, for transferring the desired polarising voltage from the measuring apparatus 50 to the capacitor 133 and for carrying out the actual measurement of the current generated by the polarographic measuring electrode system 53, the embodiment of the gated amplifier circuit 60 shown in Fig. 7 requires four transmission periods, actually the seventh, the eighth, the tenth and the eleventh transmission periods, as the determination of the input offset voltage of the operational amplifier 130 is carried out in two steps in which the first step involves the measurement of the actual voltage at the inverting input of the operational amplifier 130, and in which the second step involves the measurement of the actual voltage at the non-inverting input of the operational amplifier 130.

In the detailed diagram shown in Fig. 4, the analogue multiplexer 61 is constituted by two 8-channel multiplexers 140 and 141. The multiplexer 140 serves the purpose of transmission during the transmission periods 1-8, and the multiplexer 141 serves the purpose of transmission during the transmission periods 9-16. The individual inputs corresponding to the individual transmission periods are designated by the number of the transmission period in question. The output of the multiplexers 140 and 141 are connected to the hot terminal 90 of the circuit 23 through a resistor 147. The outputs of the multiplexers 140 and 141 are protected by an over-voltage protection circuit consisting of a resistor 143, a Zener diode 144 and two diodes 145 and 146. The diode 146 has its anode connected to the outputs of the multiplexers 140 and 141 and its cathode connected to the earth terminal 91 of the circuit 23. Consequently, the diode 146 prevents the outputs of the multiplexers 140 and 141 from shifting beyond the diode turn-on voltage, i.e. beyond 0.6-0.7 V. The resistor 143 is internally, within the circuit 23, connected to the negative supply terminal 95 and to the anode of the Zener diode 144, the cathode of which is internally connected to the earth terminal 91. The node of the resistor 143 and the anode of the Zener diode 144 are connected to the anode of the diode 145, the cathode of which is connected to the outputs of the multiplexers 140 and 141. Consequently, the outputs of the multiplexers 140 and 141 are prevented from shifting more than 0.6-0.7 V below the Zener voltage of the Zener diode 144.

The counter 56, which receives its clock pulses from the phase locked loop, voltage controlled oscillator 55, basically comprises two counter parts as indicated in Fig. 4 by the horizontal solid line of the block 56. The upper part of the counter 56 provides at its 4 outputs a binary coded representation corresponding to the division of the transmission periods into subperiods. Analogously, the lower part of the counter 56 provides at its 4 outputs a binary coded representation corresponding to the individual transmission periods. The first output of the upper part of the counter 56 corresponding to the first bit of the binary coded representation is connected to the first input of the multiplexer 140 through a resistor 142. Consequently, as mentioned above, the pulses supplied from the said first output of the counter 56 is transmitted from the multiplexer 140 of the transmitter part 10 to the receiver part 50 within the first transmission period and serves synchronizing purposes as will be explained in greater detail below. Together with the third and the fourth output of the first part of the counter 56, the said first output thereof is connected to a decoder 57 which comprises three NAND gates 152, 153 and 154, and a D flip-flop 151. As will be evident from Table 1 below, the Q output of the D flip-flop 151 is low during the subperiods Nos. 11-14. As is evident to the worker skilled in the art, the logic value present at the D input of the D flip-flop is transferred to the Q output of the flip-flop when a clock pulse is present at the CI input of the flip-flop. Caused by the inherent delay of the NAND gates 154 and 152, the shift from high to low at the D input of the D flip-flop 151 is delayed relative to the arrival of the CI clock pulse at the CI input of the D flip-flop. Consequently, the shift at the output of the flip-flop caused by the shift at the D input thereof is delayed until the succeeding shift from low to high at the CI input of the D flip-flop.

31

0153437

Table 1

| SUBPERIOD NO. | | | | | | | | | | | | | | | |
| --- | --- | --- | --- | --- | --- | --- | --- | --- | --- | --- | --- | --- | --- | --- | --- |
| 1 | 2 | 3 | 4 | 5 | 6 | 7 | 8 | 9 | 10 | 11 | 12 | 13 | 14 | 15 | 16 |
| (a) 0 | 1 | 0 | 1 | 0 | 1 | 0 | 1 | 0 | 1 | 0 | 1 | 0 | 1 | 0 | 1 |
| (b) 0 | 0 | 1 | 1 | 0 | 0 | 1 | 1 | 0 | 0 | 1 | 1 | 0 | 0 | 1 | 1 |
| (c) 0 | 0 | 0 | 0 | 1 | 1 | 1 | 1 | 0 | 0 | 0 | 0 | 1 | 1 | 1 | 1 |
| (d) 0 | 0 | 0 | 0 | 0 | 0 | 0 | 0 | 1 | 1 | 1 | 1 | 1 | 1 | 1 | 1 |
| (e) 1 | 1 | 1 | 1 | 0 | 0 | 0 | 0 | 1 | 1 | 1 | 1 | 0 | 0 | 0 | 0 |
| (f) 1 | 1 | 1 | 1 | 1 | 1 | 1 | 1 | 0 | 0 | 0 | 0 | 1 | 1 | 1 | 1 |
| (g) 1 | 0 | 1 | 0 | 1 | 0 | 1 | 0 | 1 | 0 | 1 | 0 | 1 | 0 | 1 | 0 |
| (h) 1 | 1 | 1 | 1 | 1 | 1 | 1 | 1 | 1 | 1 | 0 | 0 | 0 | 0 | 1 | 1 |

(a) = Output No. 1 of upper part of counter 56

(b) = Output No. 2 of upper part of counter 56

(c) = Output No. 3 of upper part of counter 56

(d) = Output No. 4 of upper part of counter 56

(e) = Output of NAND gate 154

(f) = Output of NAND gate 152 = D

(g) = Output of NAND gate 153 = CI

(h) = Output Q of D flip-flop 151

The lower part of the counter 56 provides at its respective outputs a binary coded representation corresponding to the division of a multiplexing cycle into 16 individual transmission periods. These outputs of the counter 56 are connected to respective addressing inputs of the decoder 58. The first 3 outputs of the lower part of the counter 56 are further connected to respective addressing inputs of the multiplexers 140 and 141. As will be evident, the said three outputs of the counter 56 provide a binary representation of the integers 1-8. In order to provide a sequential addressing of the multiplexers 140 and 141, an inhibit input of the multiplexer 140 and a corresponding inhibit input of the multiplexer 141 are connected to the output of a NAND gate 149 and the output of a NAND gate 150, respectively. The first input of the NAND gate 149 and the second input of the NAND gate 150 are connected to the Q output of the D flip-flop 151 while the first input of the NAND gate 150 is connected to the 4th output of the lower part of the counter 56. The second input of the NAND gate 149 is also connected to the 4th output of the lower part of the counter 56 through an inverter constituted by a NAND gate 148. As will be evident, the said 4th output of the lower part of the counter 56 is low within the transmission periods 1-8, and high within the transmission periods 9-16. Consequently, the second input of the NAND gate 149 is high within the transmission periods 1-8 and low within the transmission periods 9-16. The first input of the NAND gate 149 is high within the subperiods 11, 12, 13 and 14 of each transmission period. Consequently, the output of the NAND gate 149 which is connected to the inhibit input of the multiplexer 140 is low within the subperiods 11, 12, 13 and 14 of each of the transmission periods 1-8. Accordingly, the first input of the NAND gate 150 is high within the transmission periods 9-16 and, consequently, the output of the NAND gate 150 which is connected to an inhibit input of the multiplexer 141 is low within the subperiods 11, 12, 13 and 14 of the transmission periods 9-16.

At the central upper part of Fig. 4, the phase locked loop, voltage controlled oscillator 55 is shown. Basically, the voltage controlled oscillator is constituted by an integrated circuit 158 which includes a phase comparator and a voltage controlled oscillator. The oscillation frequency of the oscillator is determined by an external capacitor 159. Furthermore, the integrated circuit 158 is provided with an external RC-circuit constituted by a resistor 156 and a capacitor 157 and two external resistors 160 and 161. At its first input, the phase comparator of the integrated circuit 158 is connected to the output of a differential amplifier constituted by an operational amplifier 166. The non-inverting input of the operational amplifier 166 is connected to the anode of the Zener diode 144 which defines the maximum negative output swing from the multiplexers 140 and 141. The non-inverting input of the operational amplifier 166 is further connected to the cathode of a diode 164, the anode of which is connected to the inverting input of the operational amplifier 166. The inverting input is further connected to the cold terminal 91 constituting the earth terminal of the electronic circuit 23 through a resistor 163 and to the hot terminal 90 through the series connection of a resistor 162 and a diode 165. As is evident from Fig. 4, the diode 165 has its cathode connected to the hot terminal 90 and, consequently, only allows negative current to flow from the hot terminal 90 to the inverting input of the operational amplifier 166. When the potential at the hot terminal 90 is higher than the negative Zener reference voltage, defined by the Zener diode 144, -0.6-0.7 V, the potential at the non-inverting input of the operational amplifier 166 is 0.6-0.7 V higher than the potential at the inverting input of the operational amplifier 166, and, consequently, the output of the operational amplifier is low. When the potential at the hot terminal 90 goes below the negative Zener reference voltage -0.6-0.7 V, i.e. when a negative voltage is supplied from the receiver to the transmitter within the subperiods 1-7 of each of the transmission periods, the inverting input of the operational amplifier 166 goes below the potential at the non-inverting input of the operational amplifier, and consequently, the output of the operational amplifier 166 goes high, causing a detection of a positive transition at the first input of the phase comparator of the integrated circuit 158. This positive transition is compared to a comparator signal, mentioned above, which is input to a second input of the phase comparator of the integrated circuit 158, and which is supplied from the 4th output of the upper part of the counter 56 in an inverted state, i.e. supplied from the output of an inverter constituted by a NAND gate 155. The output of the phase comparator of the integrated circuit 158 is low pass filtered by an external low pass filter constituted by the RC network 156, 157. In case the positive transitions supplied from the output of the operational amplifier 166 and from the output of the NAND gate 155 do not coincide, the oscillation frequency of the oscillator is altered so that the time of occurrence of a positive transition at the output of the NAND gate 155 is brought to coincide with the occurrence of a positive transition at the output of the operational amplifier 166.

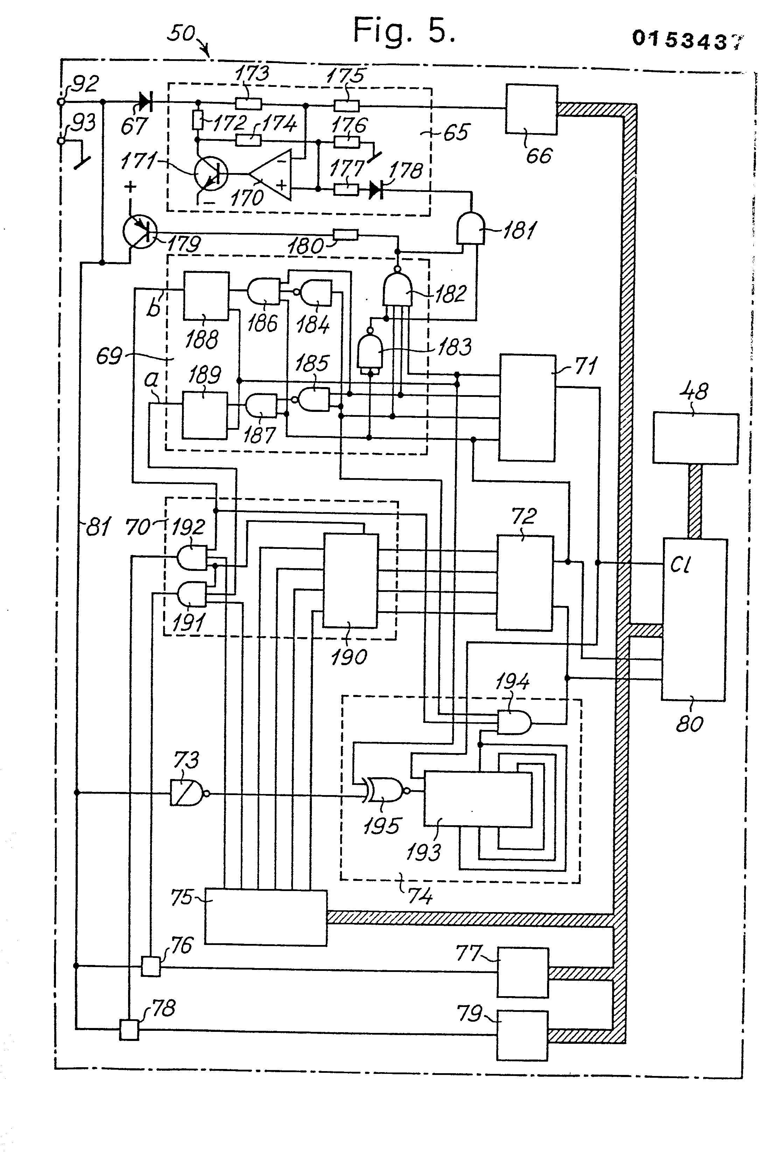

In Fig. 5, the right-hand side of the block diagram shown in Fig. 3, i.e. the receiver part of the electronic circuitry is shown. In Fig. 5, the blocks of Fig. 3 are indicated by dotted boundary lines. As explained above, the counter 71 receives its clock pulses from the microcomputer 80 and generates pulses of a frequency identical to the clock frequency, pulses of a frequency which is half the clock frequency, pulses of a frequency which is one quarter of the clock frequency and pulses of a frequency which is one eighth of the clock frequency at its outputs at the left-hand side of the block 71, from top to bottom, respectively. The outputs of the counter 71 are input to the first decoding logic 69, which includes four NAND gates 182, 183, 184 and 185, 2 AND gates 186 and 187 and two D flip-flops 188 and 189. The Q outputs of the D flip-flops 188 and 189 constitute the above b- and a-output of the decoding logic 69, respectively. In Table 2, the switching scheme of the first decoding logic 69 is shown referring to the internal outputs of the individual gates.

Table 2

| | SUB PERIOD NO. | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| (a) | 0 | 1 | 0 | 1 | 0 | 1 | 0 | 1 | 0 | 1 | 0 | 1 | 0 | 1 | 0 | 1 |
| (b) | 0 | 0 | 1 | 1 | 0 | 0 | 1 | 1 | 0 | 0 | 1 | 1 | 0 | 0 | 1 | 1 |
| (c) | 0 | 0 | 0 | 0 | 1 | 1 | 1 | 1 | 0 | 0 | 0 | 0 | 1 | 1 | 1 | 1 |
| (d) | 0 | 0 | 0 | 0 | 0 | 0 | 0 | 0 | 1 | 1 | 1 | 1 | 1 | 1 | 1 | 1 |
| (e) | 1 | 1 | 1 | 1 | 1 | 1 | 1 | 1 | 0 | 0 | 0 | 0 | 0 | 0 | 0 | 0 |
| (f) | 1 | 1 | 1 | 1 | 1 | 1 | 1 | 0 | 1 | 1 | 1 | 1 | 1 | 1 | 1 | 1 |
| (g) | 1 | 1 | 1 | 1 | 1 | 1 | 1 | 0 | 0 | 0 | 0 | 0 | 0 | 0 | 0 | 0 |
| (h) | 1 | 1 | 1 | 1 | 0 | 0 | 0 | 0 | 1 | 1 | 1 | 1 | 0 | 0 | 0 | 0 |
| (i) | 0 | 0 | 0 | 0 | 0 | 0 | 0 | 0 | 0 | 0 | 1 | 1 | 0 | 0 | 0 | 0 |
| (j) | 0 | 1 | 0 | 1 | 0 | 1 | 0 | 1 | 0 | 1 | 0 | 1 | 0 | 1 | 0 | 1 |
| (k) | 0 | 0 | 0 | 0 | 0 | 0 | 0 | 0 | 0 | 0 | 0 | 1 | 1 | 0 | 0 | 0 |
| (l) | 1 | 1 | 1 | 1 | 1 | 1 | 0 | 0 | 1 | 1 | 1 | 1 | 1 | 1 | 0 | 0 |
| (m) | 0 | 0 | 0 | 0 | 0 | 0 | 0 | 0 | 1 | 1 | 1 | 1 | 1 | 1 | 0 | 0 |
| (n) | 0 | 1 | 0 | 1 | 0 | 1 | 0 | 1 | 0 | 1 | 0 | 1 | 0 | 1 | 0 | 1 |
| (o) | 0 | 0 | 0 | 0 | 0 | 0 | 0 | 0 | 0 | 1 | 1 | 1 | 1 | 1 | 1 | 0 |

(a) =  Output No. 1 of counter 71

(b) =  Output No. 2 of counter 71

(c) =  Output No. 3 of counter 71

(d) =  Output No. 4 of counter 71

(e) =  Output of NAND gate 183

(f) =  Output of NAND gate 182

(g) =  Output of AND gate 181

(h) =  Output of NAND gate 184

(i) =  Output of AND gate 186

(j) =  CI input of D flip-flop 188

(k) = Output Q of D flip-flop 188

(l) = Output of NAND gate 185

(m) = Output of AND gate 187

(n) = Cl input of D flip-flop 189

(o) = Output Q of D flip-flop 189

As is evident from Table 2, the output Q of the D flip-flop 188 which constitutes the b-output of the decoding logic 69 is high within the 12th and 13th subperiods of each transmission period. Furthermore, the Q output of the D flip-flop 189 which constitutes the a-output of the decoding logic 69 is high within the subperiods 10-15 of each transmission period.

Through a resistor 180, the output of the NAND gate 182 is connected to a base of a pnp-transistor 179, the base collector diode of which constitutes the diode 68 shown in Fig. 3. As is evident from Table 2, the output of the NAND gate 182 is high except for the 8th subperiod of each transmission period in which the transistor 179 is turned on and, consequently, supplies a positive current to the bus bar 81 and to the hot terminal 92 and further through the coaxial cable 37 to the transmitter.

The output of the NAND gates 182 and 183 are connected to respective inputs of an AND gate 181 the output of which is connected to the current generator 65. Internally, the current generator 65 comprises a high gain differential amplifier constituted by an operational amplifier 170, the output of which is connected to the base of a npn - transistor 171 and further comprising resistors 172, 173, 174, 175, 176, and 177 and a diode 178. The resistors 173-176 are identical to one another and constitute a symmetrical resistor bridging or comparator network of the operational amplifier 170. Thus, any voltage at the output of the D/A converter 166 is inverted and transferred to the resistor 172 by means of the operational amplifier 170. Caused by this voltage drop across the resistor 172, the npn transistor 171 draws current, provided that it is turned on, through the resistor 172. Thereby, the npn transistor 171 supplies negative current determined by the voltage drop across the resistor 172 in accordance with Ohm's Law through the diode 67 to the bus bar 81 and to the hot terminal 92 and further through the coaxial cable 37 to the transmitter 10. A negative current thus generated in the current generator 65 in response to a voltage at the output of the D/A converter 66 is, as discussed above, supplied to the Zener diode 30 which is heated in accordance with the current supply thereto. As is evident from Table 2, the output of the AND gate 181 is high within the subperiods 1-7 and low within the subperiods 8-16 of each transmission period. Consequently, the logic "0" which is transferred from the output of the AND gate 181 to the non-inverting input of the operational amplifier 170 through the diode 178 and the resistor 177 shifts the output of the operational amplifier 170 low and consequently turns off the npn within the subperiods 8-16.

In the detailed diagram shown in Fig. 5, the second decoding logic 70 comprises a comparator 190 which receives a first set of inputs from the register 75 and a second set of inputs from the second counter 72. At its output, the comparator 190 provides a logic "1" in case the first set of inputs is identical to the second inputs. The output of the comparator 190 is input to an AND gate 191 and an AND gate 192 which further receive the a- and b-outputs, respectively, of the D flip-flops 189 and 188 respectively, and are further addressed from the register 75. Consequently, as explained above, the a- and b-outputs are transferred from the D flip-flops 189 and 188, respectively, through the AND gates 191 and 192, respectively, to the switches 76 and 78, respectively. As explained above, the switch 76 is activated by the a-control signal within the transmission periods Nos. 4 and 7, while within the transmission periods Nos. 2, 3, 5, 6, 8, and 9, the b-control signal is transferred to the switch 78.

The synchronization detector 74 receives an input signal from the Schmitt-trigger 73 at the input of an exclusive NOR gate 195 which also receives an input from the first output of the first counter 71. The exclusive NOR gate 195 provides a logic "0" at its output provided its inputs are not identical. The output of the exclusive NOR gate 195 is input to a cyclic counter 193 which receives its clock pulses from the clock output of the microcomputer 80. Provided that the exclusive NOR gate 195 receives a sequence "0,1,0,1" from the Schmitt-trigger 73, which is actually a threshold detecting NAND - gate, corresponding to detection of two synchronizing pulses within the first transmission period, and the exclusive NOR gate 195 at its other input receives a sequence "1,0,1,0" from the first output of the counter 71, a logic "1" is shifted through the cyclic counter 193 and output to an AND gate 194, the output of which is connected to a control input of the microcomputer 80 and to a reset-input of the counter 72. The inputs of the AND gate 194 are further connected to the b-output of the first decoding logic 69, i.e. the Q output of the D flip-flop 188 and to the third output of the first counter 71. Provided that the synchronization detector 79 detects synchronism, the AND gate 194 resets the counter 72 at the subperiod 13 of the first transmission period.

In Fig. 6, two diagrams designated A and B are shown. The diagram A illustrates schematically the voltage at the hot terminal 90 during a single transmission period. Within the subperiods 1-7 of the transmission period, the terminal 90 receives negative current of the polarity V- from the receiver, and during the subperiod No. 8, the terminal receives positive current at the polarity V+ from the receiver. During the subperiods 11, 12, 13 and 14, the multiplexer 61 is enabled, while within the subperiods 9, 10, 15 and 16, the transmitter is pending. In diagram B, a schematical time representation of the a- and b-control signals supplied from the first decoding logic 69, i.e. from the Q outputs of the D flip-flops 189, 188, respectively, is shown. When comparing the diagrams A and B, it is evident that within the transmission periods, in which the transmitter 10 acts as the actual transmitter, and the receiver 50 actually receives signals from the transmitter, the "transmitter window" shown in diagram A is larger than the "receiver window b" shown in diagram B. Analogously, in the transmission periods, in which the receiver 50 acts as the actual transmitter for transmission of compensation signals to the transmitter 10 which actually acts as a receiver, the "transmission window a" shown in diagram B is larger than the "receiver window" shown in diagram A. Consequently, the input signal occurring at the input of the actual receiver is always a signal of substantially steady state so that overshoot, instability, etc. at the input of the actual receiver is eliminated.

EXAMPLE I

In an actual laboratory realisation of the electronic circuitry shown in Figs. 3, 4 and 5, the following components were employed:

In the transmitter 10 shown in Fig. 4,

the Zener diode 30 was a Zener diode of the type BZX55 C8V2,

the pnp transistors 111 and 112 were small signal transistors of the type BC 157,

the operational amplifiers 101, 125, 130 and 166 were of the type IH 7642,

the switches 126, 127 and 132 were of the type IH 5140,

the integrated circuit 158 was of the type HEF 4046,

the integrated circuit 58 was of the type HEF 4514,

the integrated circuit 56 was of the type HEF 4020,

the integrated circuits 140 and 141 were of the type HEF 4051,

the NAND gates 148, 149, 150, 152, 153, 154 and 155 were of the type HEF 4011,

the flip-flop 151 was of the type HEF 4013,

the diodes 96, 97, 145, 146, 164 and 165 were of the type BAV 20,

the Zener diode 122 was of the type BZX 55 C2V4,

the Zener diode 144 was of the type BZX 55 C6V2,

the capacitors 98 and 99 had the value 22µF,

the capacitors 128 and 133 had the value 10nF,

the capacitor 159 had the value 100pF,

the capacitor 157 had the value 22nF,

the resistor 113 had the value 100kΩ,

the resistor 115 had the value 10kΩ,

the resistor 116 had the value 100kΩ,

the resistor 118 had the value 100kΩ,

the resistor 119 had the value 100kΩ,

the NTC resistor 51 had the value of approximately 5kΩ,

the resistor 120 had the value 5kΩ,

the NTC resistor 28 had the value of approximately 5kΩ,

the resistor 123 had the value 100kΩ,

the resistor 129 had the value 500MΩ corresponding to the internal generator impedance of the $P_{CO_2}$ electrode system,

the resistor 131 had the value 100MΩ,

the resistor 124 had the value 10kΩ,

the resistor 163 had the value 100kΩ,

the resistor 162 had the value 100kΩ,

the resistor 160 had the value 1MΩ,

the resistor 161 had the value 10kΩ,

the resistor 156 had the value 1MΩ,

the resistor 142 had the value 50kΩ,

the resistor 143 had the value 220kΩ, and

the resistor 147 had the value 1kΩ.


In the receiver 50 shown in Fig. 5,

the npn transistor 171 was of the type BD 139,

the pnp transistor 179 was of the type BC 157,

the diodes 67 and 178 were of the type BAV 20,

the operational amplifier 170 was of the type LM 324,

the AND gates 181 and 187 were of the type HEF 4081,

the NAND gate 182 was of the type HEF 4012,

the AND gates 183-185 were of the type HEF 4011,

the AND gates 186, 191, 192 and 194 were of the type HEF 4073, the Schmitt-trigger 73 was of the type HEF 40106,

the exclusive NOR gate 195 was of the type HEF 4077,

the flip-flops 188 and 189 were of the type HEF 4013,

the counters 71 and 72 were constituted by a single circuit of the type HEF 4520,

the integrated circuit 190 was of the type HEF 4585,

the integrated circuit 193 was of the type HEF 40195,

the integrated circuit 75 was of the type HEF 40173,

the switches 76 and 78 were of the type HEF 4066,

the D/A converter 77 included an integrated circuit of the type ICL 7134,

the A/D converter 79 included an integrated circuit of the type ADC 1130,

the D/A converter 66 included an integrated circuit of the type NE 5008,

the micro computer 80 was constituted by a circuit of the type Intel 8048,

the resistor 172 had the value 5Ω,

the resistors 173, 174, 175 and 176 had the value 22kΩ,

the resistor 177 had the value 1kΩ, and

the resistor 180 had the value 1.5kΩ.


In the laboratory realisation of the electronic circuitry, the leak currents of the switches 126, 127 and 131 included in the gated amplifiers 59 and 60 were in the order of 100pA.


EXAMPLE II

In a custom designed CMOS circuit realisation of the transmitter part of the electronic circuitry shown in Figs. 3, 4 and 7, the gated amplifiers 59 and 60, actually the embodiment shown in Fig. 7, and the multiplexer 61 were integrated in a first CMOS chip, and the counter and the decoders 57 and 68 were integrated into a second CMOS chip. The chips constituting the blocks 59, 60 and 61, and the blocks 56, 57 and 58, respectively, were supplied from the company AMI Micro Systems and were developed by the applicant in cooperation with the Semi-conductor Laboratory of DTH, Denmark (Laboratoriet for Halvlederteknik ved Danmarks Tekniske Højskole, Lyngby, Danmark) and the company AMI Micro Systems. The inherent characteristics of the CMOS circuitry technique and the particular layout of the chip constituting the gated amplifiers 59 and 60 and the multiplexer 61 has provided extremely small leak currents of the order of $100 \times 10^{-15}$A at a temperature of 25°C. Presently, applicant is developing a custom designed CMOS chip constituting the phase locked loop, voltage controlled oscillator 55 shown in Figs. 3 and 4.

CLAIMS

1. A method of transmission of measuring signals from an electrochemical measuring electrode device to measuring equipment, the electrode device comprising sensor means adapted to generate at least two individual measuring signals, and the measuring signals being transmitted from the electrode device to the measuring equipment through a cable, wherein, prior to transmission through the cable, the measuring signals are multiplexed in a first multiplexer means, and wherein, after transmission through the cable, the signals are demultiplexed in a second multiplexer means.

2. A method according to claim 1, wherein the measuring signals are transmitted in a time multiplexed state so that the measuring signals are transmitted from the first multiplexer means to the second multiplexer means within specific time intervals of a multiplexing cycle.

3. A method according to claim 2, wherein the time multiplexing cycle has a frequency of approximately 10 Hz-1 kHz, preferably approximately 100 Hz.

4. A method according to any of the preceding claims, wherein the measuring signals are normalized prior to multiplexing.

5. A method according to claim 4, wherein the measuring signals are normalized in offset compensated amplifier means.

6. A method according to any of the preceding claims, wherein electrical power is transmitted from said second multiplexer means to said first multiplexer means for supply thereof.

7. A method according to claim 2 or 3 and claim 6, wherein the electrical power is transmitted from said second multiplexer means to said first multiplexer means within a specific time interval of the multiplexing cycle.

8. A method according to claim 7, wherein the transmission of measuring signals from said first multiplexer means to said second multiplexer means is controlled by a voltage controlled oscillator of said first multiplexer means.

9. A method according to claim 8, wherein a synchronizing signal, which is derived from the electrical power supplied from the second multiplexer means to the first multiplexer means, and which is transmitted from the first multiplexer means to the second multiplexer means, is employed for synchronizing said first multiplexer means and said second multiplexer means relative to one another.

10. A method according to claim 2 or 3 and any of the claims 4-9, the measuring electrode device further comprising thermostating means including temperature sensor means and temperature controlling means, wherein a temperature signal representative of the temperature detected by the temperature sensor means is multiplexed in the first multiplexer means and transmitted therefrom to the second multiplexer means within a specific time period of the multiplexing cycle and is supplied to said measuring equipment, and wherein a temperature controlling signal generated in the measuring equipment, in response to the temperature representative signal, is multiplexed in the second multiplexer means and transmitted therefrom to the first multiplexer means within another specific time period of the multiplexing cycle, and supplied to the temperature controlling means for activation thereof.

11. A method according to claim 10, the measuring electrode device comprising a first and a second thermostating means including a first and a second temperature sensor means and a first and a second temperature controlling means, wherein temperature signals representative of the temperatures detected by the first and the second temperature sensor means are multiplexed in the first multiplexer means and transmitted therefrom to the second multiplexer means within a first and a second specific time period of the multiplexing cycle, respectively, and are supplied to said measuring equipment, and wherein temperature controlling signals generated in the measuring equipment, in response to the temperature representative signals, are multiplexed in the second multiplexer means and transmitted therefrom to the first multiplexer means within a third and a fourth specific time period of the multiplexing cycle and supplied to the first and the second temperature controlling means, respectively, for activation thereof.

12. A method according to claim 10 or 11, wherein the temperature controlling signal or signals transmitted from the second multiplexer means to the first multiplexer means are constituted by the current component of the power which is transmitted from the second multiplexer means to the first multiplexer means.

13. A method of transmission of at least one measuring signal from an electrochemical measuring electrode device to measuring equipment through a cable, the electrode device comprising sensor means adapted to generate said one or more measuring signals, and a first multiplexer means, and the measuring equipment comprising a second multiplexer means, wherein said one or more measuring signals are multiplexed in said first multiplexer means and are transmitted therefrom through the cable to said second multiplexer means and demultiplexed therein, and wherein an electrical power signal for supply of said electrode device is multiplexed in said second multiplexer means and is transmitted therefrom through the cable to said first multiplexer means and demultiplexed therein.

14. A method of transmission of at least one measuring signal from an electrochemical measuring electrode device which is thermostatically heated to measuring equipment through a cable, the electrode device comprising sensor means adapted to generate said one or more measuring signals, thermostating means including temperature sensor means and temperature controlling means, and a first multiplexer means, and the measuring equipment comprising a second multiplexer means, wherein said one or more measuring signals together with a temperature signal representative of the temperature detected by said temperature sensor means are multiplexed in said first multiplexer means and are transmitted therefrom through the cable to said second multiplexer means and demultiplexed therein, and wherein an electrical power signal for supply of said electrode device together with a temperature controlling signal is multiplexed in said second multiplexer means and is transmitted therefrom through the cable to said first multiplexer means and demultiplexed therein.

15. A method according to claim 13 or 14, wherein the transmission from said first multiplexer means to said second multiplexer means and vice versa is carried out in time multiplexed state so that the individual signals are transmitted within specific time intervals of a multiplexing cycle.

16. A method according to claim 15, the electrode device comprising a first and a second thermostating means including a first and a second temperature sensor means and a first and a second temperature controlling means, wherein temperature signals representative of the temperatures detected by the first and the second temperature sensor means are multiplexed in said first multiplexer means and are transmitted therefrom to said second multiplexer means within a first and a second specific time period of the multiplexing cycle, respectively, and wherein temperature controlling signals generated in the measuring equipment, in response to the temperature representative signals, are multiplexed in said second multiplexer means and are transmitted therefrom to said multiplexer means within a third and a fourth specific time period of the multiplexing cycle and supplied to the first and the second temperature controlling means, respectively.

17. A method according to claim 14 or 15, wherein the electrical power signal and said temperature controlling signal are constituted by a single signal.

18. A method according to any of the claims 15-17, wherein the time multiplexing cycle has a frequency of approximately 10 Hz-1 kHz, preferably approximately 100 Hz.

19. A method according to any of the claims 13-17, wherein said one or more measuring signals are normalized prior to multiplexing in said first multiplexer means.

0153437

20. A method according to claim 19, wherein the measuring signals are normalized in offset compensated amplifier means.

21. A method according to any of the claims 15-20, wherein the transmission of measuring signals from said first multiplexer means to said second multiplexer means is controlled by a voltage controlled oscillator of said first multiplexer means.

22. A method according to claim 21, wherein a synchronizing signal, which is derived from electrical power supplied from the second multiplexer means to the first multiplexer means, and which is transmitted from the first multiplexer means to the second multiplexer means, is employed for synchronizing said first multiplexer means and said second multiplexer means relative to one another.

23. A method according to any of the preceding claims, wherein, prior to multiplexing, the measuring signals are converted from analogue form to digital form in an A/D converter.

24. A method according to claim 23, wherein, after transmission through the cable, the measuring signals are converted from digital form to analogue form in a D/A converter.

25. A method according to any of the claims 1-22, wherein, after transmission through the cable, the measuring signals are converted from analogue form to digital form in an A/D converter prior to demultiplexing in said second multiplexer means.

26. An electrochemical measuring electrode device which comprises sensor means adapted to generate at least two individual measuring signals, and which is adapted to be connected through a cable to a corresponding measuring equipment and to cooperate therewith for transmission of said measuring signals from the electrode device to the measuring equipment, wherein the electrode device further comprises first multiplexer means which is connected to said sensor means and is adapted to provide said measuring signals, which are to be transmitted to said measuring equipment, in a multiplexed state, and to cooperate with corresponding second multiplexer means of said measuring equipment, said second multiplexer means being adapted to provide said transmitted measuring signals in a demultiplexed state.

27. An electrode device according to claim 26, wherein said multiplexer means are time multiplexer means adapted to provide said measuring signals within specific time intervals of a multiplexing cycle.

28. An electrode device according to claim 27, wherein the time multiplexing cycle of said multiplexer means has a frequency of approximately 10 Hz-1 kHz, preferable approximately 100 Hz.

29. An electrode device according to any of the claims 26-28, wherein said electrode device further comprises amplifier means interconnected between said electrode means and said first multiplexer means.

30. An electrode device according to claim 29, wherein the amplifier means are offset compensated amplifier means.

31. An electrode device according to claim 30, wherein the offset compensated amplifier means is constituted by a gated amplifier means.

32. An electrode device according to any of the claims 26-31, wherein said electrode device further comprises internal power supply means adapted to receive power from said measuring equipment.

33. An electrode device according to any of the claims 27-32, wherein the electrode device further comprises an oscillator adapted to determine the frequency of the time multiplexing cycle.

34. An electrode device according to claim 33, wherein said oscillator is a voltage controlled oscillator, the oscillation frequency of which is determined by the voltage supplied from said internal power supply means.

35. An electrode device according to any of the claims 26-34, wherein the sensor means is constituted by a potentiometric electrode system and a polarographic electrode system.

36. An electrode device according to any of the claims 26-35, wherein the electrode device further comprises thermostating means including temperature sensor means and temperature controlling means, wherein the temperature sensor means and the temperature controlling means are connected to said first multiplexer means for transmission of a signal representative of the temperature detected by the temperature sensor means from said first multiplexer means to said second multiplexer means of the measuring equipment and for transmission of a temperature controlling signal generated in the measuring equipment, in response to the temperature representative signal, from said second multiplexer means to said first multiplexer means, and wherein said first multiplexer means is further adapted to supply said temperature controlling signal to said temperature controlling means for activation thereof.

37. An electrode device according to claim 35 and 36 being an electrochemical measuring electrode device for transcutaneous measurement of the partial pressures of $O_2$ and $CO_2$.

38. An electrochemical measuring electrode device which comprises at least one sensor means adapted to generate at least one measuring signal, and which is adapted to be connected through a cable to corresponding measuring equipment and to cooperate therewith for transmission of said one or more measuring signals from the electrode device to the measuring equipment, wherein the electrode device further comprises first multiplexer means connected to said sensor means and adapted to cooperate with corresponding second multiplexer means of said measuring equipment, wherein said first multiplexer means is adapted to provide said one or more measuring signals to be transmitted to said measuring equipment in a multiplexed state, said second multiplexer means being adapted to provide said transmitted one or more measuring signals in a demultiplexed state, wherein said first multiplexer means is further adapted to receive an electrical power supply signal for supply of the electrode device from said second multiplexer means, and wherein said first multiplexer means is further adapted to supply said electrical power supply signal to the electrode device.

39. A thermostatically heated electrochemical measuring electrode device which comprises at least one sensor means adapted to generate at least one measuring signal and thermostating means including temperature sensor means and temperature controlling means, and which is adapted to be connected through a cable to corresponding measuring equipment and to cooperate therewith for transmission of said one or more measuring signals from the electrode device to the measuring equipment, wherein the electrode device further comprises first multiplexer means connected to said sensor means and adapted to cooperate with corresponding second multiplexer means of said measuring equipment, wherein said first multiplexer means is adapted to provide said one or more measuring signal or signals to be transmitted to said measuring equipment in a multiplexed state, said second multiplexer means being adapted to provide said transmitted one or more measuring signals in a demultiplexed state, wherein said first multiplexer means is further connected to the temperature sensor means and the temperature controlling means, and is adapted to transmit a signal representative of the temperature detected by the temperature sensor means to said second multiplexer means of the measuring equipment and to receive an electrical power supply signal for supply of the electrode device and a temperature controlling signal generated in the measuring equipment, in response to the temperature representative signal, from said second multiplexer means, and wherein said first multiplexer means is further adapted to supply said electrical power supply signal to the electrode device and to supply said temperature controlling signal to said temperature controlling means for activation thereof.

40. An electrode device according to claim 38 or 39, wherein said multiplexer means are time multiplexer means and are adapted to transmit said signals within specific time intervals of a multiplexing cycle.

41. An electrode device according to claim 40, wherein the time multiplexing cycle of said multiplexer means has a frequency of approximately 10 Hz-1 kHz, preferably approximately 100 Hz.

42. An electrode device according to any of the claims 38-41, wherein said electrode device further comprises amplifier means interconnected between said electrode means and said first multiplexer means.

43. An electrode device according to claim 42, wherein the amplifier means are offset compensated amplifier means.

44. An electrode device according to claim 42, wherein the offset compensated amplifier means is constituted by a gated amplifier means.

45. An electrode device according to any of the claims 40-42, wherein the electrode device further comprises an oscillator adapted to determine the frequency of the time multiplexing cycle.

46. An electrode device according to any of the claims 40-45, wherein said oscillator is a voltage controlled oscillator, the oscillation frequency of which is determined by the voltage supplied from said internal power supply means.

47. An electrode device according to any of the claims 40-46, wherein the first multiplexer means is adapted to transmit a synchronizing signal which is derived from the electrical power supply signal supplied from the second multiplexer means to the first multiplexer means, and which is transmitted from the first multiplexer means to the second multiplexer means within a specific transmission period of the multiplexing cycle for synchronizing said first multiplexer means and said second multiplexer means relative to one another.

48. An electrode device according to any of the claims 38-47, wherein the sensor means comprises a potentiometric electrode system.

49. An electrode device according to any of the claims 38-48, wherein the sensor means comprises a polarographic electrode system.

50. An electrode device according to any of the claims 26-49 being an electrochemical measuring electrode device for transcutaneous measurement of blood gas partial pressures.

51. An electrode device according to any of the claims 26-50, wherein the electronic circuitry is constituted by an integrated, custom designed circuit.

52. A gated amplifier means for connection to an external measuring signal generating source and to external measuring equipment, comprising
a high-gain differential DC-amplifier having an output, an inverting input and a non-inverting input,
a storage capacitor connected to one of the inputs of the DC-amplifier and to a DC-supply source for supply of an input offset compensation voltage to the said one input of the DC-amplifier, and
a switch means for conversion of the gated amplifier means from a first mode in which an input signal supplied from the signal generating source is amplified in the DC-amplifier and supplied from the output thereof to the measuring equipment, to a second mode in which any input offset of the DC-amplifier is transmitted in a unity gain mode from the gated amplifier means to the measuring equipment and vice versa.

53. A gated amplifier means according to claim 52, wherein the switch means comprises a first switch interconnected between the output of the DC-amplifier and the inverting input thereof for converting the DC-amplifier into a unity gain mode, when short-circuiting the output and the inverting input of the DC-amplifier, for transmitting any input offset of the DC-amplifier therethrough and further from the output thereof to the external measuring equipment.

54. A gated amplifier means according to claim 52 or 53, wherein the DC-amplifier is connected in a non-inverting mode, and wherein the

0153437

switch means comprises a second switch interconnected between the signal generating source and the non-inverting input of the DC-amplifier, and a third switch interconnected between the storage capacitor and the non-inverting input of the DC-amplifier.

55. A gated amplifier means according to claim 52, comprising a further high-gain differential DC-amplifier connected in a non-inverting mode, the output of said further DC-amplifier being connected to said external measuring equipment, and the switch means comprising a first and a second switch, the first switch being interconnected between the non-inverting input of the further DC-amplifier and the inverting input of the first-mentioned DC-amplifier, and the second switch means being interconnected between the non-inverting input of the further DC-amplifier and the non-inverting input of the first-mentioned DC-amplifier, for transmitting, when the first switch and the second switch, respectively, are activated, any input offset of the inverting input of the first-mentioned DC-amplifier and any input offset of the non-inverting input of the first-mentioned DC-amplifier, respectivly to the further DC-amplifier and further therethrough in a unity-gain, non-inverting mode to the external measuring equipment.

56. A gated amplifier means according to any of the claims 52-55, wherein the DC-amplifier is constituted by an operational amplifier.

57. An electrochemical measuring system comprising an electrochemical measuring electrode device and a corresponding measuring equipment having any of the characteristics of the above claims.

Fig. 1.

Fig. 2.

0153437

Fig. 3.

0153437

Fig. 4.

0153437

Fig. 5.

0153437

## Fig. 6

0153437

## Fig. 7

## CLAIMS INCURRING FEES

The present European patent application comprised at the time of filing more than ten claims.

☐ All claims fees have been paid within the prescribed time limit. The present European search report has been drawn up for all claims

☐ Only part of the claims fees have been paid within the prescribed time limit. The present European search report has been drawn up for the first ten claims and for those claims for which claims fees have been paid,

namely claims

☐ No claims fees have been paid within the prescribed time limit. The present European search report has been drawn up for the first ten claims

## LACK OF UNITY OF INVENTION

The Search Division considers that the present European patent application does not comply with the requirement of unity of invention and relates to several inventions or groups of inventions,
namely:

```
Claims 1-51, 57: A method and a device for transmission
of measuring signals
Claims 52-56: A gated amplifier
```

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims:     1-51,57

## DOCUMENTS CONSIDERED TO BE RELEVANT

| Category | Citation of document with indication. where appropriate, of relevant passages | Relevant to claim | CLASSIFICATION OF THE APPLICATION (Int. Cl.) |
|---|---|---|---|
| A | US - A - 3 962 697 (VREELAND) <br><br> -- | 1-3,23 24,26- 28,57 | G 08 C 15/00 <br> A 61 B 5/00 <br> G 01 N 27/28 <br> H 03 F 3/72 |
| Y | US - A - 3 876 997 (FARLEY et al.) <br><br> * Figures 1,2 * <br><br> -- | 25 | |
| A | GB - A - 1 391 948 (RICHALET) <br><br><br> -- | 1-3,23, 24,26- 28,57 | |
| X | US - A - 3 910 257 (FLETCHER et al) <br><br> * Figures 1,2 * | 1-3,23, 24,26- 29,57 | TECHNICAL FIELDS SEARCHED (Int. Cl.) |
| Y | <br><br> -- | 6-7,10 17,25, 32,36, 38-42 | G 08 C <br> A 61 B <br> G 01 N |
| Y | US - A - 4 334 541 (LEST et al.) <br><br> * Column 2, line 39 - column 3, line 6 * <br><br> -- | 10-12, 14-17, 36,39 | |
| X | US - A - 3 925 762 (HEITLINGER et al.) <br><br> * Column 3, line 50 - column 4, line 21 ; column 5, lines 61-66; | 1-3,23, 24,26- | |

The present search report has been drawn up for all claims

| Place of search | Date of completion of the search | Examiner |
|---|---|---|
| STOCKHOLM | 02-08-1984 | LANDSTRÖM |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.) |
|---|---|---|---|
| Y | claim 11; figure 1* | 29,57 6,7,10- 17,25, 32,36, 38-42 | |
| | -- | | |
| Y | DE - A - 3 232 009 (MEDTRONIC INC.) | | |
| | * Abstract * | 6-7,13, 15,32, 38,40- 42 | |
| | -- | | |
| A | DE - A - 1 908 652 (LICENTIA PATENT-VERWALTUNGS GmbH) | | |
| | * The whole document * | 1-3,26- 29,57 | |
| | -- | | **TECHNICAL FIELDS SEARCHED (Int. Cl.)** |
| X | US - A - 3 665 439 (BRUMMER et al) | | |
| | * Figure 1 * | 1-4,26- 29,57 | |
| Y | | 6,7,10- 17,25, 32,36, 38-42 | |
| | ------- | | |

The present search report has been drawn up for all claims

| Place of search | Date of completion of the search | Examiner |
|---|---|---|
| | | |

EPO Form 1503. 03 82